# EUROPEAN PATENT APPLICATION

(11) **EP 4 484 428 A1**
(43) Date of publication of application: **01.01.2025**
(21) Application number: 23306073.0
(22) Date of filing: 29.06.2023
(51) Int. Cl.: C07D 487/00, C07B 59/00, A61K 31/395, A61P 35/00

(54) **CUCURBITURIL DERIVATIVES FOR RADIOLABELING, PROCESSES OF PRODUCTION AND USES THEREOF**

(71) Applicant: Université de Bourgogne, 21000 Dijon (FR); Centre National de la Recherche Scientifique, 75016 Paris (FR)
(72) Inventor: GONCALVES, Victor, 21000 DIJON (FR); VIZIER, Romane, 21000 DIJON (FR)
(74) Representative: Gevers & Orès

(57) **Abstract**

The present invention concerns cucurbituril derivatives for radiolabeling, processes of production and uses thereof. Said invention also concerns the corresponding radiolabeled cucurbituril derivatives, processes of production and uses thereof to obtain conjugates by supramolecular chemistry, in particular with targeting agents, more particularly labelled biomolecules, notably in nuclear medicine.

## Description

The present invention concerns cucurbituril derivatives for radiolabeling, processes of production and uses thereof. Said invention also concerns the corresponding radiolabeled cucurbituril derivatives, processes of production and uses thereof to obtain conjugates by supramolecular chemistry, in particular with targeting agents, more particularly labelled biomolecules, notably in nuclear medicine.

Antibodies, notably monoclonal antibodies, but also engineered affinity proteins, such as Affibody molecules and Affitins, and antibody fragments such as Fab, F(ab')₂, VHH fragments, and VNAR fragments, and constructs such as minibodies, diabodies, and singlechain variable region fragments (scFvs), owing to their outstanding affinity and selectivity for their target antigens, are vectors of choice for the targeting of cells and tissues of interest in vivo. They are successfully used, after radiolabeling, in nuclear medicine for nuclear imaging by positron emission tomography (PET) or single photon emission tomography (SPECT), or for targeted internal radiation therapy (TIR).

Positron emission tomography (PET) is a functional imaging technique that uses radioactive substances known as radiotracers to visualize and measure changes in metabolic processes, and in other physiological activities including blood flow, regional chemical composition, and absorption.

Single-photon emission computed tomography (SPECT) is a nuclear medicine tomographic imaging technique using gamma rays. It is similar to conventional nuclear medicine planar imaging using a gamma camera (scintigraphy), but is able to provide true 3D information. This information is typically presented as cross-sectional slices through the patient, but can be freely reformatted or manipulated as required. The technique generally needs delivery of a gamma-emitting radioisotope (a radionuclide) into the patient, normally through injection into the bloodstream.

Targeted internal radiation therapy (TIR, also named Targeted Radionuclide Therapy, or Molecular Radionuclide Therapy) is a form of radiation therapy used in interventional radiology to treat cancer. It is generally for selected patients with surgically unresectable cancers, for example hepatocellular carcinoma or metastasis to the liver.

Radiometalation of a biomolecule, such as a protein, traditionally involves two steps: first, the conjugation of a bifunctional chelator to the biomolecule, and second, the coordination of a radiometal by the biomolecule-chelator conjugate. The chelator ensures the rapid and stable formation of metal complexes.

The chelators are often built around a polyamine system, more particularly polyazamacrocyclic motif, allowing the formation of complexes with good in vivo stability with a wide variety of clinically important radiometals. This is, for instance, the case of DOTA (1,4,7,10-tetraazacyclododecane-1,4,7,10-tetraacetic acid). The radiometalation step frequently requires heating to achieve satisfactory complexation yields. While this is not usually a problem with small molecules or peptides, it becomes far more critical when it comes to labelling proteins.

This issue motivated the development of a novel approach to protein radiolabeling: rather than trying to radiolabel chelator-antibody bioconjugates, one solution would be to first prepare the radiometal-chelator complex, under optimal conditions to obtain high radiochemical yields, and then to graft this radiolabeled complex to the protein under mild conditions, compatible with the stability of these biomolecules. This is classically achieved by creating covalent bonds between protein residues and the radiometal-chelator complex. Such approach requires the presence of a reactive function on the chelator, that will react with another function present on the biomolecule. For instance, the reaction between an N-hydroxysuccidimyl ester functionalized radiometal-chelator complex and the amine functions of lysine residues, creates an amide bond. Alternatively, bioorthogonal reactive functions, such as azide, trans-cyclooctenes, tetrazines or cycloctynes, can be used to perform so-called "click reactions", which are high yielding, fast and selective, even in vivo. However, these reactive functions inherently exhibit a certain degree of instability. For example, N-hydroxysuccidimyl esters are slowly hydrolyzed in water. Similarly, tetrazines and trans-cyclooctenes have been shown to undergo degradation reactions resulting in loss of functionality.

An alternative to covalent coupling is the implementation of non-covalent conjugation strategies, by supramolecular assembly. This type of approach has been used, for example, to label an antibody carrying a biotin, with [¹¹¹In]In-DTPA-(strept)avidin. Biotin forms extremely stable interactions with (strept)avidin. Despite its exceptional affinity (K = 10¹⁵ M⁻¹), the use of the biotin-(strept)avidin pair suffers from the immunogenicity of (strept)avidin and its large size, which can have a substantial impact on the properties of the conjugate.

There is a need for the development of new tools for the preparation of radiopharmaceuticals, for use in nuclear medicine (for instance by PET or SPECT imaging), or for TIR therapy.

Accordingly, it is an object of the present invention to provide new compounds, with excellent chemical stability, good water solubility and of limited molecular weight, which can be easily radiolabeled, with high efficiency with a wide variety of metals of interest, and that can be conjugated to biomolecules, under mild conditions, in order to preserve the bioactivity of said biomolecules.

Large biomolecules, such as monoclonal antibodies, circulate for several days in the body before reaching their target and being eliminated from the organism. Thus, the administration of radiolabelled proteins to the patient is responsible for the irradiation of multiple healthy organs until the radiopharmaceutical is eliminated. The dose received by radiosensitive organs (dose-limiting organs) such as bone marrow, kidney or liver may limit the amount of radioactivity that can be administered to patients.

The pretargeting approach, first proposed in 1985 by Meares et al, addresses this problem: a radioactive agent is administered to the patient only after the targeting biomolecule, carrying a tag, has had sufficient time to accumulate in the tumour and has been cleared from healthy organs. The radioactive agent recognizes the tag of the tumorbound vector in vivo, which enables the selective accumulation of radioactivity in the tumor. Unbound radioactive agent is rapidly cleared from the body. Thus, this approach is an excellent strategy to reduce side effects and increase the therapeutic window of radi opharmaceuti cals.

The reaction between the radioactive agent and the tagged biomolecule needs to be rapid, under physiological conditions, and selective. For instance, "copper-free click reactions" such as the inverse-electron demand Diels-Alder reaction are currently being investigated for this type of application. However, the reactive partners (in particular transcyclooctene) suffer from instability issues. The radioactive agent should ideally be chemically stable, non-immunogenic, and have rapid biodistribution in the body, with limited accumulation in healthy organs, and preferentially a urinary excretion.

Therefore, another aim of the present invention is to provide new radiolabeled compounds that have advantageous hydrophilicity, aqueous solubility, biodistribution properties and biocompatibility, serving as valuable tools for the construction of radioconjugates for nuclear medicine through pretargeting approaches. In sharp contrast with the invention, more hydrophobic radioactive probes may accumulate in the liver, and intestines, and be eliminated through a hepatobiliary route. This may lead to a risk of overirradiation of these organs and may interfere with the detection of tissues of interest (e.g. tumor, metastases) in these organs.

Thus, the present invention relates to a compound of following formula (I): 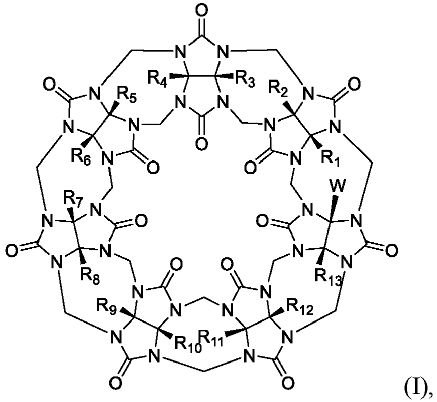 wherein:
W is of following formula (A):

   -X-L-(Y)ᵢ-Z (A),
R₁, R₂, R₃, R₄, R₅, R₆, R₇, R₈, R₉, R₁₀, R₁₁ and R₁₂ are identical and are:
   - H;
   - OH; or
   - a methyl group;
R₁₃ is:
   - H;
   - OH;
   - a (C₁-C₆) alkyl group
   - a aryl group, in particular a phenyl; or
   - a O-(C₁-C₁₂)-alkyl, O-(C₂-C₁₂)-alkenyl, or O-(C₂-C₁₂)-alkynyl group, said group being optionally interrupted by at least one atom or group chosen from -O-, -NR-, -S-, and/or substituted, notably terminally substituted, by a T group chosen from halogens, in particular -Cl; ORₐ; and NRₐR_{b}; Rₐ and R_{b} being independently chosen from Hand (C₁-C₁₂)-alkyl groups;
i is at each occurrence independently chosen from 0 and 1;
X is chosen from O, CH₂, NHC(=O), and NH;
L is a linear or branched (C₁-C₂₄)-alcane diyl group, optionally interrupted by at least one atom or group chosen from -O-, -NR-, -S-, -C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-; -C(R)=C(R')-, -C=C-, and arene diyl, in particular benzene diyl, or by a group of the following formula: 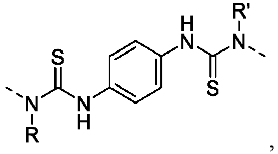
Y is chosen from -O-, -NR-, -S-, -C(=O)-, -O-C(=O)-, -C(=O)-O-, -NR-C(=O)-, - C(=O)-NR-, -NR-C(=O)-NR'-, -NR-C(=S)-NR'-, -NR-C(=O)-O-, -O-C(=O)-NR'-; - C(R)=C(R')-, -C=C-, arene diyl, in particular benzene diyl, and groups of one of the following formulae: 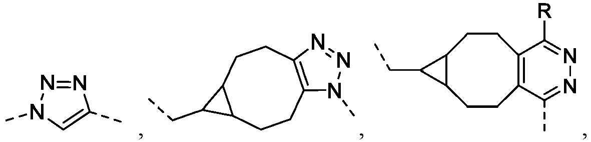 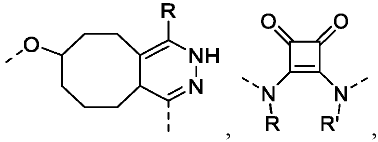 in particular 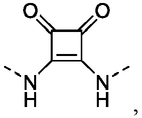
R and R' are at each occurrence independently chosen from H and C₁-C₆-alkyl groups;
Z is a chelator.

By a group of one of the following formulae: 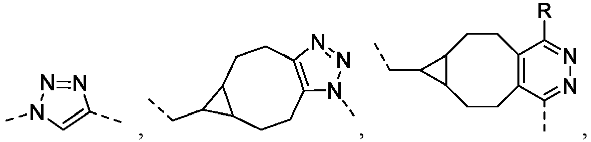 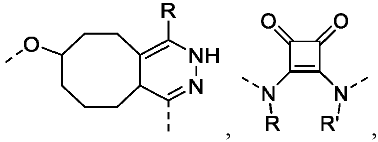 in particular 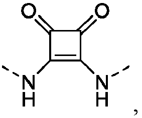 is notably meant a group of one of the following formulae: 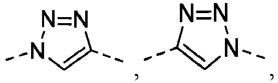 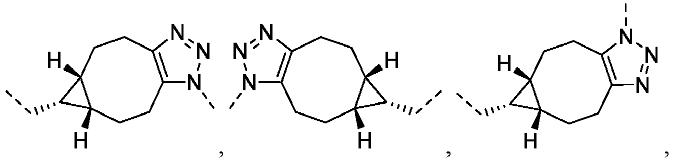 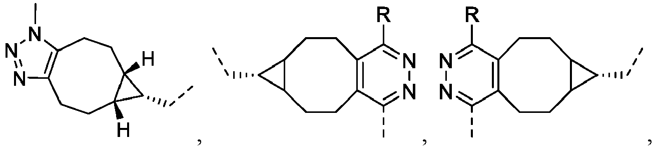 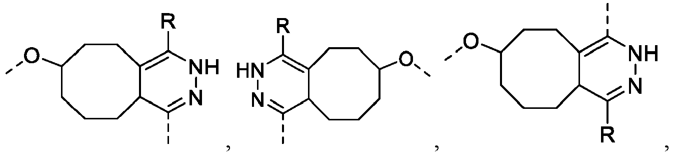 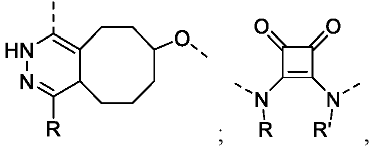 in particular 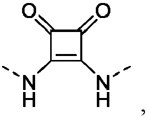 and their stereoisomers.

In a particular embodiment, the present invention relates to a compound of formula (I), wherein:
W is of following formula (A):

   -X-L-(Y)ᵢ-Z (A),
R₁, R₂, R₃, R₄, R₅, R₆, R₇, R₈, R₉, R₁₀, R₁₁ and R₁₂ are identical and are:
   - H; or
   - OH;
R₁₃ is:
   - H;
   - OH; or
   - a (C₁-C₆) alkyl group;
i is at each occurrence independently chosen from 0 and 1;
X is chosen from O, CH₂, and NHC(=O);
L is a linear or branched (C₁-C₂₄)-alcane diyl group, optionally interrupted by at least one atom or group chosen from -O-, -NR-, -S-, -C(=O)-, -O-C(=O)-, -C(=O)-O-, -NR-C(=O)-, -C(=O)-NR-, -NR-C(=O)-NR'-, -NR-C(=S)-NR'-NR-C(=O)-O-, -O-C(=O)-NR'-; -C(R)=C(R')-, -C=C-, and arene diyl, in particular benzene diyl, or by a group of the following formula: 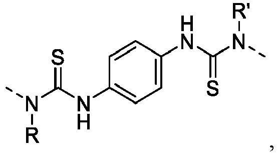
Y is chosen from -O-, -NR-, -S-, -C(=O)-, -O-C(=O)-, -C(=O)-O-, -NR-C(=O)-, - C(=O)-NR-, -NR-C(=O)-NR'-, -NR-C(=S)-NR'-, -NR-C(=O)-O-, -O-C(=O)-NR'-; - C(R)=C(R')-,
   -C=C-, arene diyl, in particular benzene diyl, and groups of one of the following formulae: 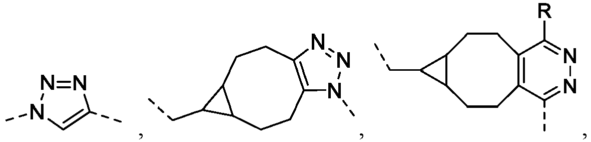 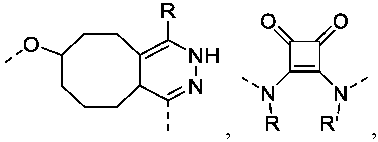 in particular 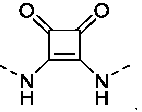
R and R' are at each occurrence independently chosen from H and C₁-C₆-alkyl groups;
Z is a chelator.

In a particular embodiment, all of the R₁-R₁₃ groups are H.

In a particular embodiment, R₁₃ group is not H, R₁₃ being in particular OH or a (C₁-C₆) alkyl group, the R₁-R₁₂ groups being H.

In a particular embodiment, L is at each occurrence independently a linear (C₁-C₁₂)-alcane diyl group, in particular a linear (C₁-C₆)-alcane diyl group, more particularly a C₂-alcane diyl group.

In a particular embodiment, L is a (C₁-C₆)-alcane diyl group, in particular a C₂-alcane diyl group, i is 1 and Y is NH orNH-C(=O); or L₁ is a (C₁-C₆)-alcane diyl group, in particular a C₂-alcane diyl group, i is 1, j is 0, and Y is NH or NH-C(=O).

In a particular embodiment, W is of following formula (A₁): 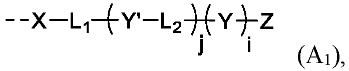 wherein:
X, Y, Z, and i are as defined above;
j is at each occurrence independently chosen from 0 and 1;
L₁ and L₂ are at each occurrence independently a linear or branched (C₁-C₁₂)-alcane diyl group, optionally interrupted by at least one atom or group chosen from -O-, -NR-, -S-, -C(R)=C(R')-, -C=C-, and arene diyl, in particular benzene diyl;
Y' is chosen from -C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-; -C(R)=C(R')-, -C≡C-, arene diyl, in particular benzene diyl, and by groups of one of the following formulae: 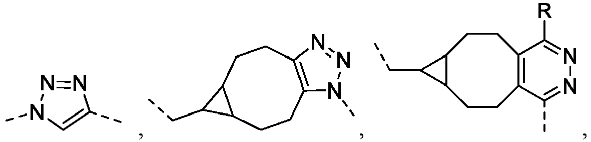 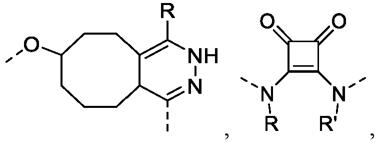 in particular 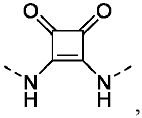 R and R' are at each occurrence independently chosen from Hand C₁-C₆-alkyl groups.

In a particular embodiment, the present invention concerns a compound as defined above, being of the following formula (Ia):

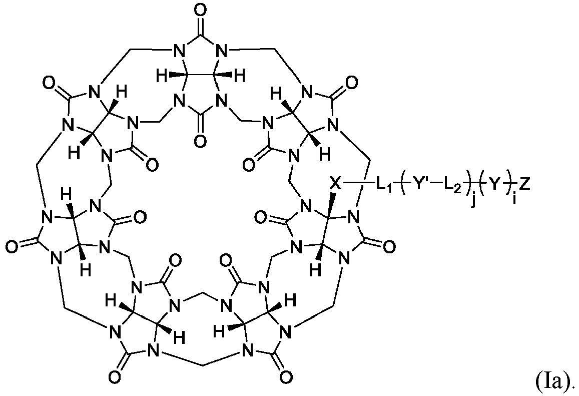

In a particular embodiment, W is of one of the following formulae: 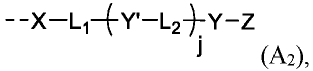 - -X-L₁-Y'-L₂-Z (A₃), --X-L₁-Y'-L₂-Y-Z _{(A4),} --X-L₁₋Z _{(A5),} --X-L₁₋Y-Z _{(A6),} wherein X, Y, Y', Z, L₁, L₂ and j are as defined above.

In a particular embodiment, X is O.

In a particular embodiment, L₁ and/or L₂ are at each occurrence independently a linear (C₁-C₁₂)-alcane diyl group, in particular a linear (C₁-C₆)-alcane diyl group, optionally interrupted by at least one atom -O-.

In a particular embodiment, L₁ and/or L₂ are at each occurrence independently a linear (C₁-C₁₂)-alcane diyl group, in particular a linear (C₁-C₆)-alcane diyl group, more particularly a C₂-alcane diyl group.

In a particular embodiment, L₁ and/or L₂ are at each occurrence independently a - (CH₂-CH₂-O)ₙ-CH₂-CH₂- group, wherein n ranges from 1 to 7, notably from 1 to 5.

In a particular embodiment, i is 1 and Y is NH, NHC(=O) or -C(=O)-NH.

In a particular embodiment, j is 1 and Y' is NHC(=O) or -C(=O)-NH.

In a particular embodiment, L₁ is a (C₁-C₆)-alcane diyl group, in particular a C₂-alcane diyl group, i is 0, j is 1, Y' is NHC(=O) or -C(=O)-NH, and L₂ is a (C₁-C₆)-alcane diyl group, in particular a C₂-alcane diyl group.

In a particular embodiment, Z is chosen from DOTA (tetraxetan or 1,4,7,10-tetraazacyclododecane-1,4,7,10-tetraacetic acid), NODAGA (1,4,7-triazacyclononane,1-glutaric acid-4,7-acetic acid), DOTAGA (1,4,7,10-tetraazacyclododececane,1-(glutaric acid)-4,7,10-triacetic acid), DTPA (diethylenetriaminepentaacetic acid), DFO (desferrioxamine), NOTA (1,4,7-Triazacyclononane-1,4,7-triacetic acid), RESCA (2,2'-(((1S,2S)-2-((carboxymethyl)(4-(2-oxo-2 (perfluorophenoxy)ethyl)benzyl)amino)cyclohexyl)azanediyl)diacetic acid), NODA-MPAA (1,4,7-triazacyclononane- 1,4-diacetate-methyl phenylacetic acid), HBED (bis(2-hydroxybenzyl) ethylenediaminediacetic acid), TETA (1,4,8,11-tetraazacyclododecane-1,4,8,11-tetraacetic acid), CB-TE2A (4,11-bis-(carboxymethyl)-1,4,8,11-tetraazabicyclo[6.6.2]-hexadecane), Macropa (N,N'-bis[(6-carboxy-2-pyridyl)methyl]-4,1 3-diaza-18-crown), HOPO (octadentate hydroxypyridinone type group of chelators), TRAP (3-({4,7-bis-[(2-carboxy-ethyl)-hydroxy-phosphinoylmethyl]-[1,4,7]triazonan-1-ylmethyl} -hydroxy-phosphinoyl)-propionic acid), THP (hexadentate tris(3,4-hydroxypyridinone)), DATA ([4-carboxymethyl-6-(carboxymethyl-methyl-amino)-6-methyl-[1,4]diazepan-1-yl] -acetic acid), NOTP (1,4,7-triazacyclononane-N,N',N"-tris(methylene phosphonic)acid)), sarcophagine (3,6,10,13,16,19-hexaazabicyclo[6.6.6]icosane), FSC (3,15,27-triamino-7, 19,31-trihydroxy-10,22,34-trimethyl-1,13,25-trioxa-7,19,31-triaza-cyclohexatriaconta-9,21,33-triene-2,8,14,20,26,32-hexaone), NETA ({4-[2-(bis-carboxymethyl-amino)-ethyl]-7-carboxymethyl-[1,4,7]triazonan-1-yl}-acetic acid), H4octapa (N,N-(6-carboxy-2-pyridylmethyl)-N,N-diacetic acid-1,2-diaminoethane), Pycup (1,8-(2,6-pyridinedimethylene)-1,4,8,11-tetraazacyclo-tetradecane), NₓS₄₋ₓ (N4, N2S2, N3S) (a group of tetradentate chelators with N-atoms (basic amine or non-basic amide) and thiols as donors stabilizing Tc-complexes, especially Tc(V)- oxo complexes), Hynic (6-hydrazino-nicotinic acid), ^{99m}Tc(CO)₃-chelators (bi- or tridendate chelators capable of forming stable complexes with technetium tricarbonyl fragments), and as exemplified as follows:

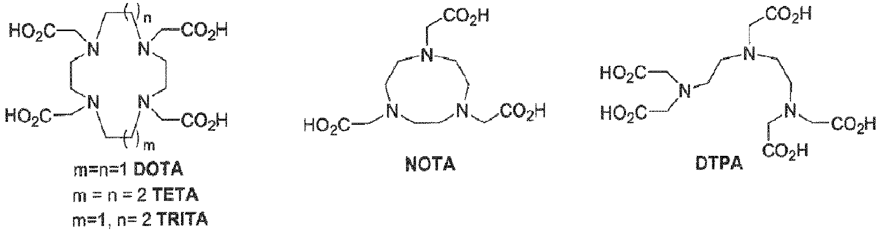

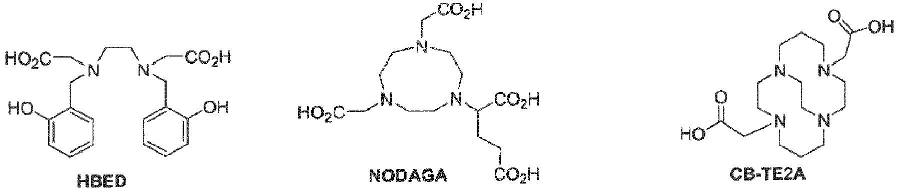

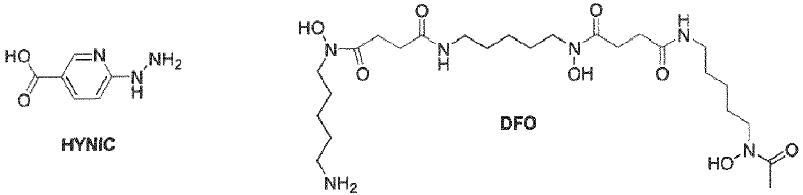

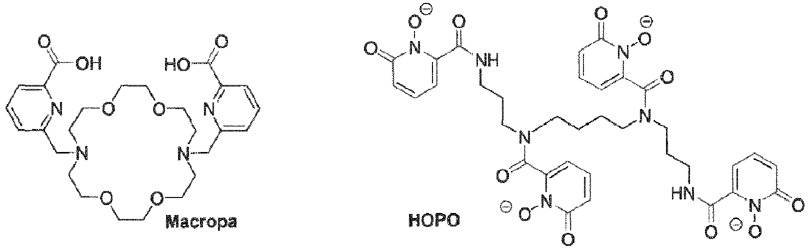

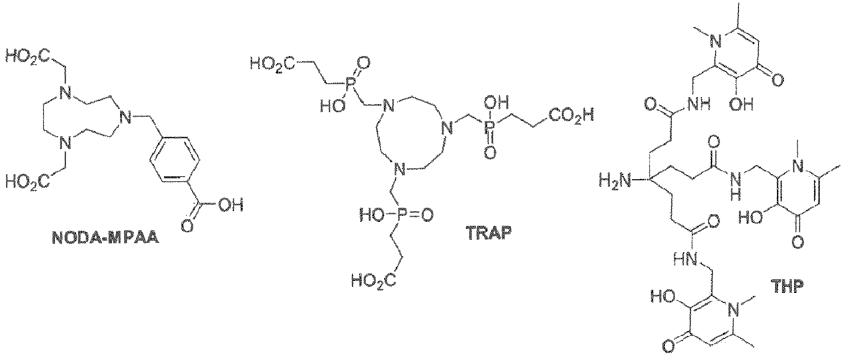

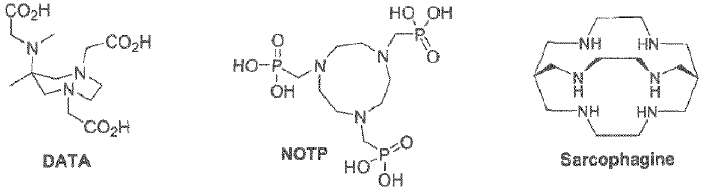

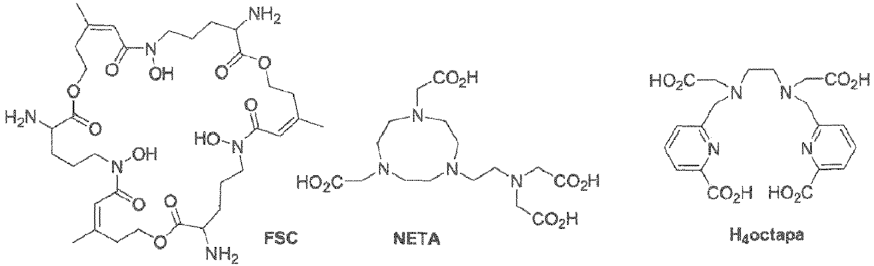

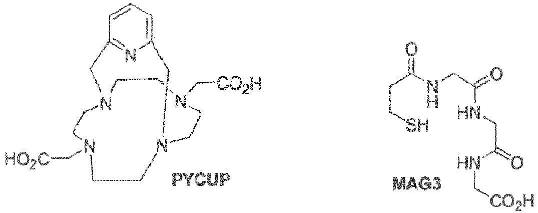

In this context, the Y group may for example be a NH residue forming with an acid group of the chelator an amide bond, or may not be present, the linker L, L₁ or L₂ being directly bound to a carbon atom of the chelator.

In a particular embodiment, -L-(Y)ᵢ-Z is chosen from the following formulae, X being in particular O:

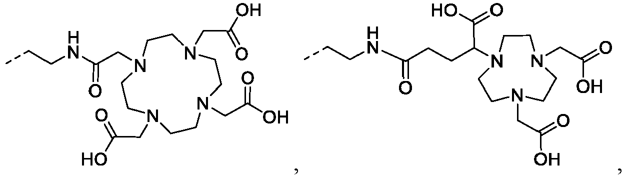

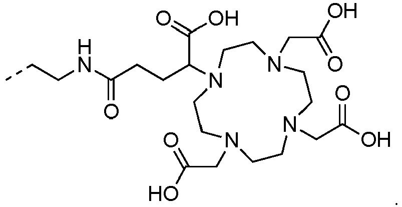

In a particular embodiment, the present invention concerns a compound as defined above being chosen from the following formulae:

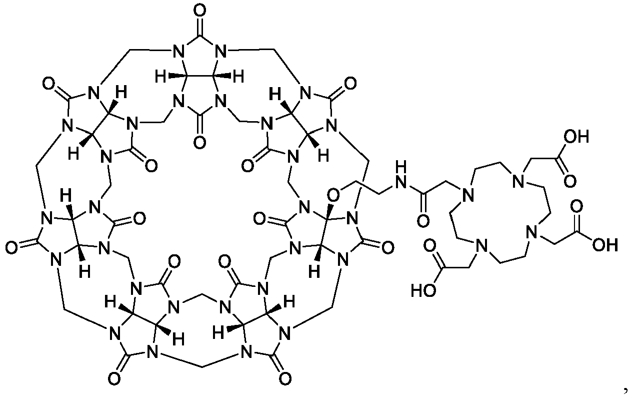

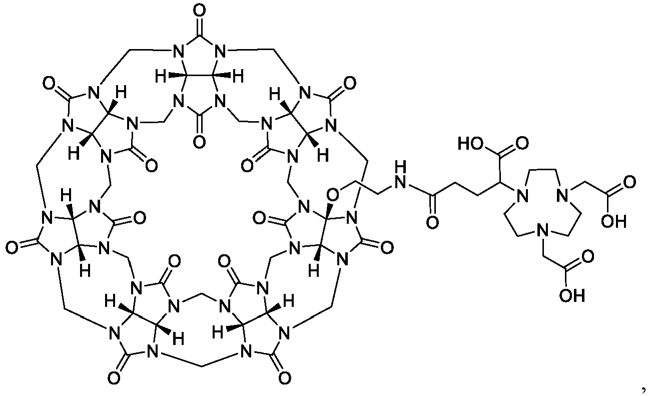

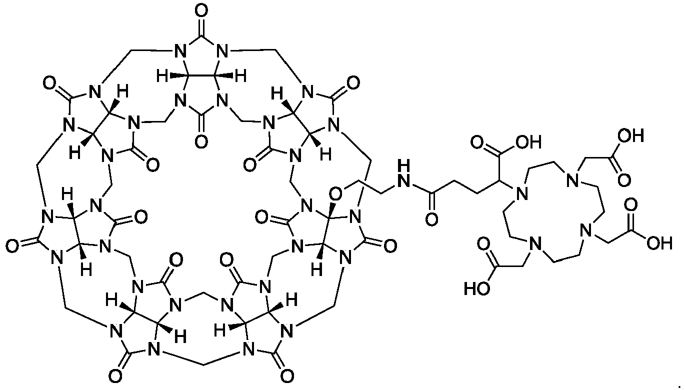

Compounds of the invention may be obtained as follows.

When X is O, and R₁-R₁₃ are H, compounds of formula (I) may be prepared according to the following scheme: 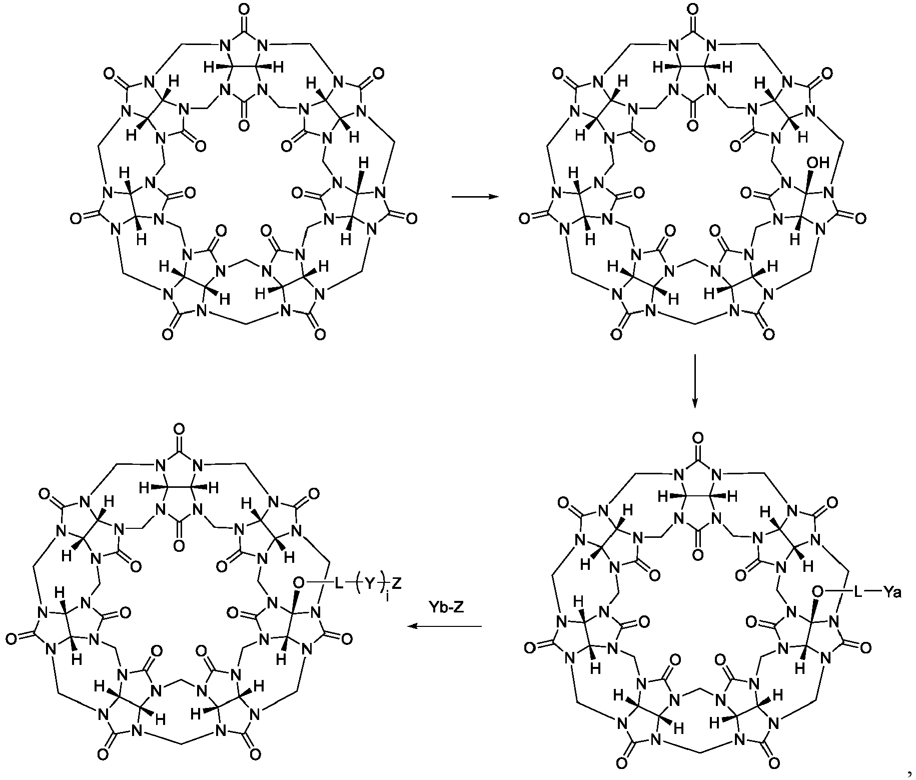 wherein Yₐ and Y_{b} are groups, well known from the skilled in the art, that enable the formation of the group Y (or the Z group in case the Y group is included in the final Z group), by methods well known from the skilled in the art as well.

In the case R₁-R₁₃ = OH, the compounds of the invention can be obtained by peroxidation of the cucurbit[7]uril (CB[7]) starting material, with R₁-R₁₃ = H. An OH function can then randomly be modified by alkylation (for example with NaH, then R-X, where X is a leaving group).

When R₁₃ is not H, R₁₃ being for example Me, compounds of formula (I) may be prepared according to the following scheme: 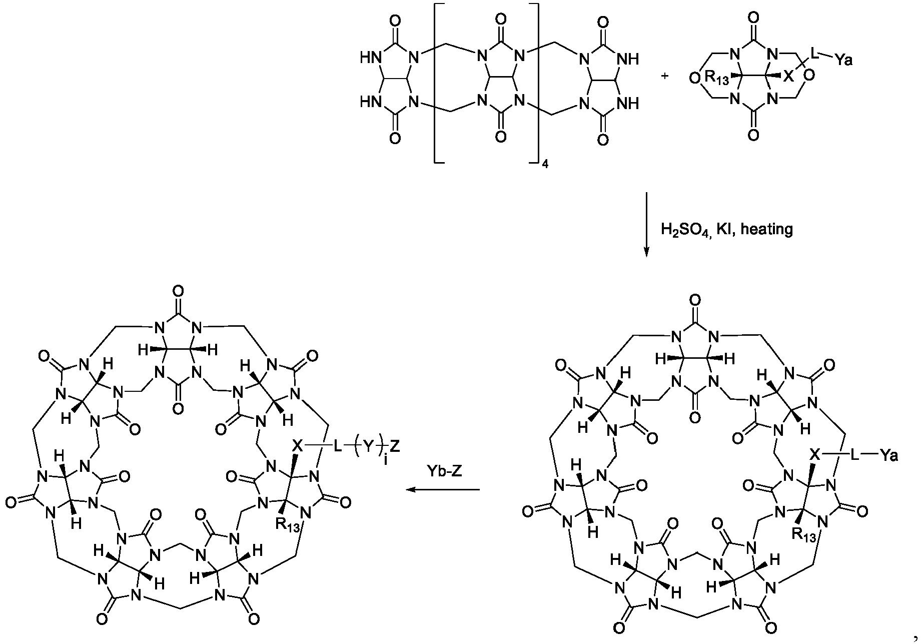 and for example: 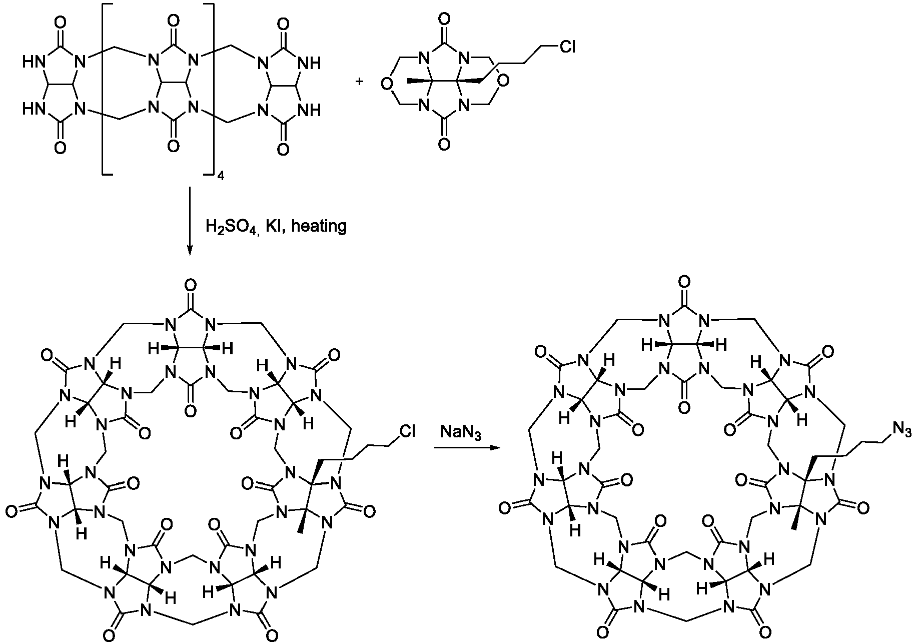 wherein Yₐ and Y_{b} are groups, well known from the skilled in the art, that enable the formation of the group Y (or the Z group in case the Y group is included in the final Z group), by methods well known from the skilled in the art as well.

The above-mentioned reactions may for example be performed analogously to what has been described in J. Am. Chem. Soc. 2012, 134, 31, 13133-13140.

For R₁-R₁₂ = methyl group, the synthesis can be obtained through the above-mentioned strategy, from the appropriate glycoluril hexamer.

In particular, compounds of formula (Ia) may be prepared according to one of the following schemes: 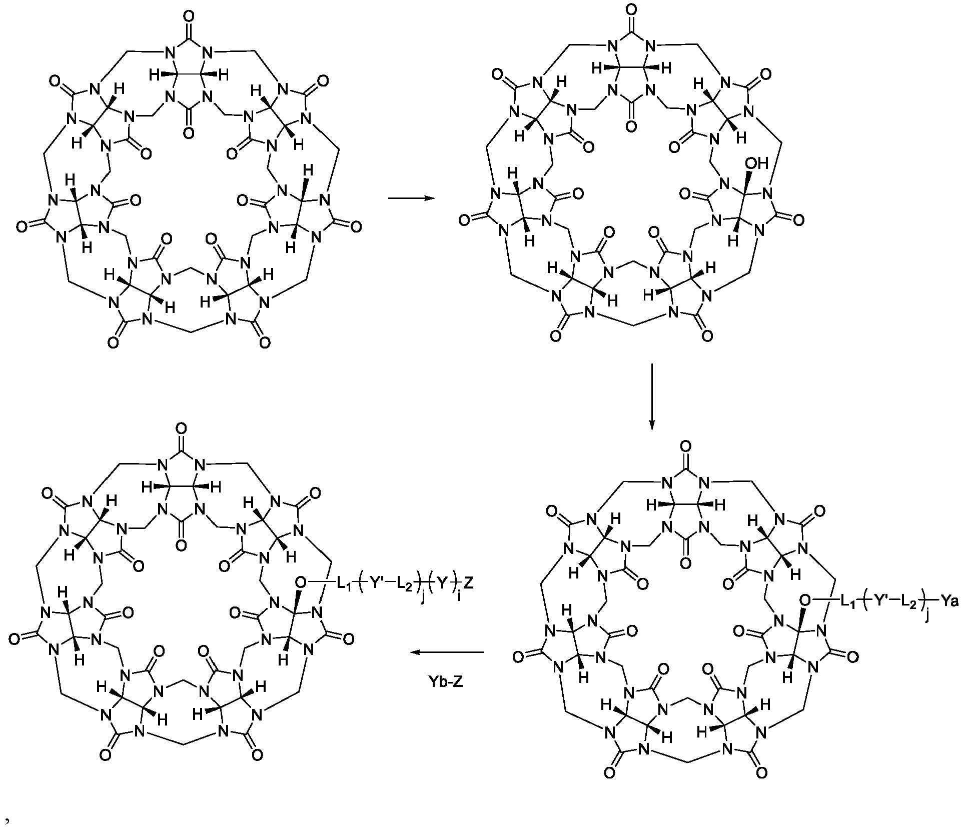 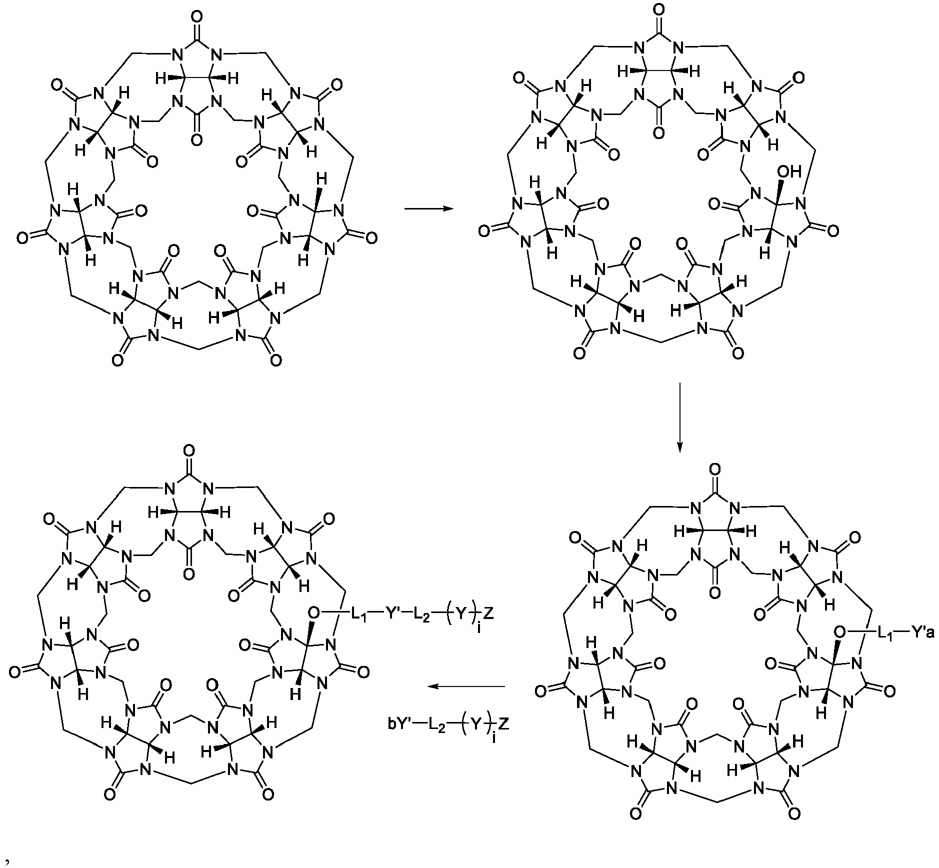 wherein Y'ₐ and Y'_{b} are groups, well known from the skilled in the art, that enable the formation of the group Y', by methods well known from the skilled in the art as well, and wherein Yₐ and Y_{b} are groups, well known from the skilled in the art, that enable the formation of the group Y (or the Z group in case the Y group is included in the final Z group), by methods well known from the skilled in the art as well.

In another aspect, the present invention also concerns a compound of following formula (II): 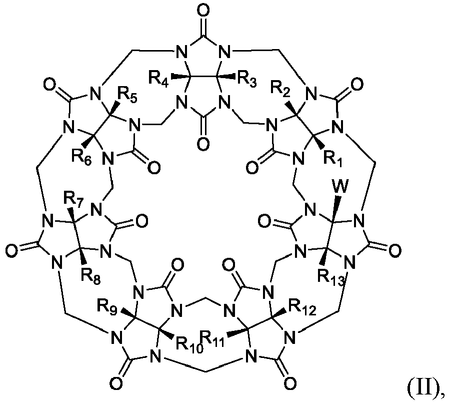 wherein:
W is of following formula (B):

   --X-L-(Y)ᵢ-ZM (B),
R₁, R₂, R₃, R₄, R₅, R₆, R₇, R₈, R₉, R₁₀, R₁₁ and R₁₂ are identical and are:
   - H;
   - OH; or
   - a methyl group;
R₁₃ is:
   - H;
   - OH;
   - a (C₁-C₆) alkyl group
   - a aryl group, in particular a phenyl;
   - a O-(C₁-C₁₂)-alkyl, O-(C₂-C₁₂)-alkenyl, or O-(C₂-C₁₂)-alkynyl group, said group being optionally interrupted by at least one atom or group chosen from -O-, -NR-, -S-, and/or substituted, notably terminally substituted, by a T group chosen from halogens, in particular -Cl; ORₐ; and NRₐR_{b}; Rₐ and R_{b} being independently chosen from Hand (C₁-C₁₂)-alkyl groups;
i is at each occurrence independently chosen from 0 and 1;
X is chosen from O, CH₂, NHC(=O), and NH;
L is a linear or branched (C₁-C₂₄)-alcane diyl group, optionally interrupted by at least one atom or group chosen from -O-, -NR-, -S-, -C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-; -C(R)=C(R')-, -C=C-, and arene diyl, in particular benzene diyl, or by a group of the following formula: 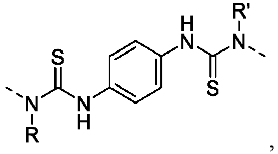
Y is chosen from -O-, -NR-, -S-, -C(=O)-, -O-C(=O)-, -C(=O)-O-, -NR-C(=O)-, - C(=O)-NR-, -NR-C(=O)-NR'-, -NR-C(=S)-NR'-, -NR-C(=O)-O-, -O-C(=O)-NR'-; - C(R)=C(R')-,
   -C=C-, arene diyl, in particular benzene diyl, and groups of one of the following formulae: 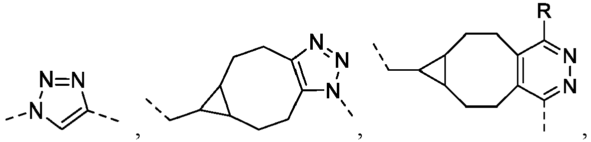 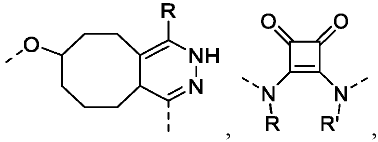 in particular 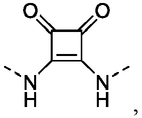
R and R' are at each occurrence independently chosen from H and C₁-C₆-alkyl groups;
Z is a chelator;
M is a radiometal.

All the embodiments defined above in the framework of the compound of formula (I) may also apply here, alone or in any combination.

In a particular embodiment, M is chosen from ⁴³Sc, ⁴⁴Sc, ⁴⁷Sc, ⁵¹Cr, ⁶²Cu, ⁶⁴Cu, ⁶⁷Cu, ⁶⁷Ga, ⁶⁸Ga, ^{81m}Kr, ⁸²Rb, ⁸⁹Zr, ⁹⁰Y, ^{99m}Tc, ¹¹¹In, ^{117m}Sn, ¹⁴⁹Tb, ¹⁵²Tb, ¹⁵³Sm, ¹⁵⁵Tb, ¹⁶¹Tb, ¹⁶⁶Ho, ¹⁷⁷Lu, ¹⁸⁶Re, ¹⁸⁸Re, ²⁰¹Tl, ²⁰³Pb, ²¹²Bi, ²¹²Pb, ²¹³Bi, ²²³Ra, ²²⁵Ac, ²²⁷Th.

In a more particular embodiment, M is chosen from ⁴³Sc, ⁴⁴Sc, ⁵¹Cr, ⁶²Cu, ⁶⁴Cu, ⁶⁷Ga, ⁶⁸Ga, ^{81m}Kr, ⁸²Rb, ⁸⁹Zr, ^{99m}Tc, ¹¹¹In, ^{117m}Sn, ¹⁵²Tb, ¹⁵⁵Tb, ¹⁶⁶Ho, ²⁰¹Tl, ²⁰³Pb. In this case, the compounds of the invention may be used for TEP and/or SPECT imaging.

In another more particular embodiment, M is chosen from ⁴⁷Sc, ⁶⁷Cu, ⁸⁹Sr, ⁹⁰Y, ¹⁴⁹Tb, ¹⁵³Sm, ¹⁶¹Tb, ¹⁶⁶Ho, ¹⁷⁷Lu, ¹⁸⁶Re, ¹⁸⁸Re, ²¹²Bi, ²¹²Pb, ²¹³Bi, ²²³Ra, ²²⁵Ac, ²²⁷Th. In this case, the compounds of the invention may be used as therapeutics.

In another aspect, the present invention also concerns a conjugate between at least one compound of following formula (II) as defined above and at least one compound (III) comprising a targeting agent, in particular biomolecule, residue linked, optionally through a linker L', to a binder of cucurbit[7]urils.

The present invention notably concerns a conjugate between at least one compound of following formula (II) as defined above and a compound (III) comprising a targeting agent, in particular biomolecule, residue linked, optionally through a linker L', to a binder of cucurbit[7]urils.

The present invention notably concerns a conjugate between a compound of following formula (II) as defined above and a compound (III) comprising a targeting agent, in particular biomolecule, residue linked, optionally through a linker L', to a binder of cucurbit[7]urils.

The present invention notably concerns a conjugate between at least two, in particular two, three or four, more particularly two, compounds of following formula (II) as defined above and a compound (III) comprising a targeting agent, in particular biomolecule, residue linked, optionally through a linker L', to a binder of cucurbit[7]urils.

All the embodiments defined above in the framework of the compound of formula (I) or (II) may also apply here, alone or in any combination.

In a particular embodiment, the targeting agent is chosen from biomolecules, nanoparticles, and liposomes.

The term "biomolecule" refers in particular to any natural biological molecule or any synthetically derived molecule that mimics or is an analogue of a natural biological molecule. Biomolecules include polynucleotides (e.g. RNA, DNA) such as nucleic acid aptamers, peptides, polypeptides, proteins, antibodies, polysaccharides, lipids. The amino acids constituting said peptides, polypeptides, and proteins may include amino acids other than those found in the 20 naturally occurring amino acids. These amino acids may also include non-proteinogenic functional groups (e.g. CF₃, N₃, F, NO₂). The term "polysaccharides" includes mono-, di- and oligosaccharides comprising O- and N- glycosyl linkages. Polysaccharides may include functional groups that are not commonly found in a cellular environment (e.g. cyclopropene, halogens and nitriles). Lipids include amphipathic, phospho- and glycolic lipids and sterols such as cholesterol. An "amphipathic lipid" refers to a lipid with both hydrophilic and hydrophobic characteristics. A "phospholipid" refers to a lipid bound to a phosphate group and carrying a charge. Examples of phospholipids include phosphatidic acid, phosphatidylethanolamine, phosphatidylcholine, phosphatidylserine and phosphatidylinositol. A "glycolipid" is a lipid bound to a poly- or oligosaccharide. Examples of glycolipids include galactolipids, sulfolipids, glycosphingolipids and glycosylphosphatidylinositol. Lipids may include substituents rarely found in the cellular environment (e.g. cyclopropene, halogens and nitriles).

In a more particular embodiment, the biomolecule is selected from the group comprising an antibody, a peptide, a peptidomimetic, a protein, a nucleic acid aptamer.

In an even more particular embodiment, the biomolecule is an antibody, in particular a human, humanized, or chimeric antibody.

In another even more particular embodiment, the biomolecule is an IgG1, IgG2, or IgG4 antibody.

In another even more particular embodiment, the biomolecule is an antibody, in particular a monoclonal antibody, more particularly chosen from monoclonal antibodies targeting proteins expressed at the surface of cancer cells or in the tumor microenvironment. The cancer is in particular thyroid cancer, neuroendocrine tumour, lung cancer, notably small-cell lung cancer, breast cancer, prostate cancer, notably castration-resistant prostate cancer, bone cancer, in particular bone metastases, non-Hodgkin lymphoma, notably large-cell lymphoma such as low-grade non-Hodgkin lymphoma, transformed low-grade non-Hodgkin lymphoma, diffuse large B-cell non-Hodgkin lymphoma, follicular lymphoma, notably follicular large-cell lymphoma, relapsed non-Hodgkin lymphoma, refractory low-grade non-Hodgkin lymphoma, transformed B-cell non-Hodgkin lymphoma, indolent non-Hodgkin lymphoma, pheochromocytoma, paraganglioma, neuroblastoma, CNS cancer, in particular CNS metastases, small-round-cell tumour, pancreatic cancer, in particular pancreatic ductal adenocarcinoma, colorectal cancer, gastric cancer, head and neck cancer, multiple myeloma, lymphoma, liver cancer, in particular hepatocellular carcinoma, liver metastases, visceral metastases, gastroenteropancreatic neuroendocrine tumours.

Said antibody is for example chosen from atezolizumab, avelumab, bevacizumab, cemiplimab, cetuximab, dinutuximab, durvalumab, elotuzumab, ipilimumab, isatuximab, mogamulizumab, necitumumab, nivolumab, Obinutuzumab, ofatumumab, olaratumab, panitumumab, pembrolizumab, pertuzumab, ramucirumab, rituximab, trastuzumab.

In a particular embodiment, the binder of cucurbit[7]urils is a ferrocene or a polycycloalkane, in particular an adamantane, bicyclo[2.2.2]octane, or a diamantane, said ferrocene or polycycloalkane substituted by at least one amine -NR_{c}R_{d}, ammonium - N⁺R_{c}R_{d}Rₑ, linear or branched (C₁-C₂)-alkyl-NR_{c}R_{d}, or linear or branched (C₁-C₂)-alkyl-N⁺R_{c}R_{d}Rₑ, wherein R_{c}, R_{d} and Rₑ are independently chosen from H and (C₁-C₆)-alkyl groups, and optionally further substituted by at least one hydroxyl, linear or branched (C₁-C₆)-alkyl, linear or branched (C₁-C₆)-alkyl-OH.

In a particular embodiment, the linker L' is a linear or branched (C₁-C₂₄)-alcane diyl group, optionally interrupted by at least one atom or group chosen from -O-, -NR-, -S-, - C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-; -C(R)=C(R')-, -C=C-, and arene diyl, in particular benzene diyl, or by a group of the following formula:

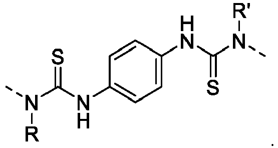

Said linker L' may be linked to said targeting agent by a Y" group chosen from -O-, -NR-, -S-, -C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-; -C(R)=C(R')-, and -C=C-, or by a group of one of the following formulae: 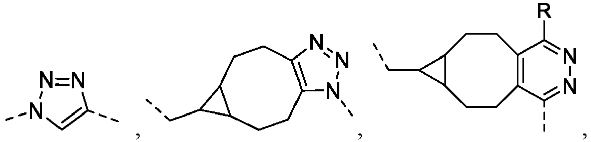 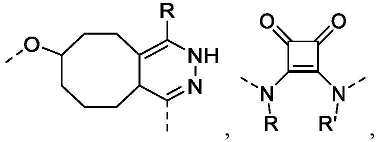 in particular 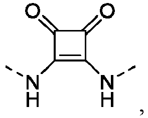
R and R' are at each occurrence independently chosen from H and C₁-C₆-alkyl groups.
L' is for example a PEG.

In another aspect, the present invention also concerns a diagnostic and/or therapeutic composition, comprising a compound of formula (II) as defined above, and a pharmaceutically acceptable excipient.

All the embodiments defined above in the framework of the compound of formula (I) or (II) may also apply here, alone or in any combination.

In another aspect, the present invention also concerns the compound of formula (II) as defined above for use as diagnostic or therapeutic agent for detecting, diagnosing or treating cancer, in particular thyroid cancer, neuroendocrine tumour, lung cancer, notably small-cell lung cancer, breast cancer, prostate cancer, notably castration-resistant prostate cancer, bone cancer, in particular bone metastases, non-Hodgkin lymphoma, notably large-cell lymphoma such as low-grade non-Hodgkin lymphoma, transformed low-grade non-Hodgkin lymphoma, diffuse large B-cell non-Hodgkin lymphoma, follicular lymphoma, notably follicular large-cell lymphoma, relapsed non-Hodgkin lymphoma, refractory low-grade non-Hodgkin lymphoma, transformed B-cell non-Hodgkin lymphoma, indolent non-Hodgkin lymphoma, pheochromocytoma, paraganglioma, neuroblastoma, CNS cancer, in particular CNS metastases, small-round-cell tumour, pancreatic cancer, in particular pancreatic ductal adenocarcinoma, colorectal cancer, gastric cancer, head and neck cancer, multiple myeloma, lymphoma, liver cancer, in particular hepatocellular carcinoma, liver metastases, visceral metastases, gastroenteropancreatic neuroendocrine tumours.

All the embodiments defined above in the framework of the compound of formula (I) or (II) may also apply here, alone or in any combination.

In another aspect, the present invention also concerns a combination of a compound of formula (II) as defined above, with a compound of formula (III) as defined above, for use in the diagnosis or treatment of cancer, in particular thyroid cancer, neuroendocrine tumour, lung cancer, notably small-cell lung cancer, breast cancer, prostate cancer, notably castration-resistant prostate cancer, bone cancer, in particular bone metastases, non-Hodgkin lymphoma, notably large-cell lymphoma such as low-grade non-Hodgkin lymphoma, transformed low-grade non-Hodgkin lymphoma, diffuse large B-cell non-Hodgkin lymphoma, follicular lymphoma, notably follicular large-cell lymphoma, relapsed non-Hodgkin lymphoma, refractory low-grade non-Hodgkin lymphoma, transformed B-cell non-Hodgkin lymphoma, indolent non-Hodgkin lymphoma, pheochromocytoma, paraganglioma, neuroblastoma, CNS cancer, in particular CNS metastases, small-round-cell tumour, pancreatic cancer, in particular pancreatic ductal adenocarcinoma, colorectal cancer, gastric cancer, head and neck cancer, multiple myeloma, lymphoma, liver cancer, in particular hepatocellular carcinoma, liver metastases, visceral metastases, gastroenteropancreatic neuroendocrine tumours, with simultaneous, separate or spread out administration over time.

All the embodiments defined above in the framework of the compound of formula (I), (II) or (III) may also apply here, alone or in any combination.

In another aspect, the present invention also concerns the conjugated compound as defined above, for use in the diagnosis or treatment of cancer, in particular thyroid cancer, neuroendocrine tumour, lung cancer, notably small-cell lung cancer, breast cancer, prostate cancer, notably castration-resistant prostate cancer, bone cancer, in particular bone metastases, non-Hodgkin lymphoma, notably large-cell lymphoma such as low-grade non-Hodgkin lymphoma, transformed low-grade non-Hodgkin lymphoma, diffuse large B-cell non-Hodgkin lymphoma, follicular lymphoma, notably follicular large-cell lymphoma, relapsed non-Hodgkin lymphoma, refractory low-grade non-Hodgkin lymphoma, transformed B-cell non-Hodgkin lymphoma, indolent non-Hodgkin lymphoma, pheochromocytoma, paraganglioma, neuroblastoma, CNS cancer, in particular CNS metastases, small-round-cell tumour, pancreatic cancer, in particular pancreatic ductal adenocarcinoma, colorectal cancer, gastric cancer, head and neck cancer, multiple myeloma, lymphoma, liver cancer, in particular hepatocellular carcinoma, liver metastases, visceral metastases, gastroenteropancreatic neuroendocrine tumours.

All the embodiments defined above in the framework of the compound of formula (I), (II) or (III) may also apply here, alone or in any combination.

In another aspect, the present invention also concerns a method of treating cancer in a subject, the method comprising administering to a subject in need thereof a pharmaceutically effective amount of a compound of formula (II) as defined above.

In another aspect, the present invention also concerns a method of diagnosing cancer in a subject, the method comprising administering to a subject in need thereof a diagnostically effective amount of a compound of formula (II) as defined above.

### DEFINITIONS

The following terms and expressions contained herein are defined as follows:
As used herein, a range of values in the form "x-y" or "x to y", or "x through y", include integers x, y, and the integers there between. For example, the phrases "1-6", or "1 to 6" or "1 through 6" are intended to include the integers 1, 2, 3, 4, 5, and 6. Preferred embodiments include each individual integer in the range, as well as any subcombination of integers. For example, preferred integers for "1-6" can include 1, 2, 3, 4, 5, 6, 1-2, 1-3, 1-4, 1-5, 2-3, 2-4, 2-5, 2-6, etc.

As used herein, the term "patient" or "subject" refers to a warm blooded animal such as a mammal, preferably a human, or a human child, which is afflicted with, or has the potential to be afflicted with one or more diseases and conditions described herein.

For the purpose of the invention, the term "pharmaceutically acceptable" is intended to mean what is useful to the preparation of a pharmaceutical composition, and what is generally safe and non-toxic, for a pharmaceutical use.

The term "pharmaceutically acceptable salt or solvate" is intended to mean, in the framework of the present invention, a salt or solvate of a compound which is pharmaceutically acceptable, as defined above, and which possesses the pharmacological activity of the corresponding compound.

The pharmaceutically acceptable salts comprise:
(1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and
(2) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

Acceptable solvates for the therapeutic use of the compounds of the present invention include conventional solvates such as those formed during the last step of the preparation of the compounds of the invention due to the presence of solvents. As an example, mention may be made of solvates due to the presence of water (these solvates are also called hydrates) or ethanol.

It is recognized that compounds of the present invention may exist in various stereoisomeric forms. As such, the compounds of the present invention include both diastereomers and enantiomers. The compounds are normally prepared as racemates and can conveniently be used as such, but individual enantiomers can be isolated or synthesized by conventional techniques if so desired. Such racemates and individual enantiomers and mixtures thereof form part of the present invention.

It is well known in the art how to prepare and isolate such optically active forms. Specific stereoisomers can be prepared by stereospecific synthesis using enantiomerically pure or enantiomerically enriched starting materials. The specific stereoisomers of either starting materials or products can be resolved and recovered by techniques known in the art, such as resolution of racemic forms, normal, reverse-phase, and chiral chromatography, recrystallization, enzymatic resolution, or fractional recrystallization of addition salts formed by reagents used for that purpose. Useful methods of resolving and recovering specific stereoisomers described in Eliel, E. L.; Wilen, S.H. Stereochemistry of Organic Compounds; Wiley: New York, 1994, and Jacques, J, et al. Enantiomers, Racemates, and Resolutions; Wiley: New York, 1981, each incorporated by reference herein in their entireties.

It is also specified that all the formulae defined in the present specification include all the possible resonance structures, being in particular noted that the structures represented herein may not be the most stable resonance structures.

The term "alkyl", as used in the present invention, refers in particular to a straight or branched saturated hydrocarbon chain, or a saturated or partially saturated mono- or bicyclic alkyl ring system.

Unless stated otherwise, the term "alkyl" thus includes the term "cycloalkyl", which refers in particular to a saturated or partially saturated mono- or bicyclic alkyl ring system.

The term "(C₁-C_{X})alkyl", as used in the present invention, refers in particular to a straight or branched saturated hydrocarbon chain containing from 1 to X carbon atoms.

For instance, the term "(C₁-C₇)alkyl", includes, but is not limited to, methyl, ethyl, n-propyl, iso-propyl, n-butyl, iso-butyl, sec-butyl, t-butyl, n-pentyl, n-hexyl, n-heptly and the like, or a (C₃-C₇)cycloalkyl, such as cyclopropyl, cyclobutyl, cyclopentyl, cyclohexyl, cycloheptyl, cyclooctyl, pinenyl, adamantanyl and the like.

The term "alcoxy" as used in the present invention, refers in particular to alkyl-O-group, wherein the term "alkyl" is as defined above.

As used herein, the term "aryl" refers to a substituted or unsubstituted, mono- or bicyclic hydrocarbon aromatic ring system having 6 to 10 ring carbon atoms. Examples include phenyl and naphthyl. Preferred aryl groups include unsubstituted or substituted phenyl and naphthyl groups. Included within the definition of "aryl" are fused ring systems, including, for example, ring systems in which an aromatic ring is fused to a cycloalkyl ring. Examples of such fused ring systems include, for example, indane, indene, and tetrahydronaphthalene.

The term "halogen", as used in the present invention, refers in particular to a bromine, chlorine or iodine atom.

The term "alkenyl" refers in particular to any straight or branched hydrocarbon chain, carrying at least one double bond, of 2 to 12 carbon atoms, preferably 2 to 6 carbon atoms, such as for example ethenyl, 2-propenyl, 2-butenyl, 3-butenyl, 2-pentenyl and its isomers, 2-hexenyl and its isomers, 2,4-pentadienyl.

The term "alkynyl" refers in particular to any linear or branched hydrocarbon chain, carrying at least one triple bond, of 2 to 12 carbon atoms, preferably of 2 to 6 carbon atoms, such as for example ethynyl, 2-propynyl, 2-butynyl, 3-butynyl, 2-pentynyl and its isomers, 2-hexynyl and its isomers

The term "chelating agent" refers in particular to a polydentate molecule capable of forming coordination bonds with a metal ion to form a metal complex, also known as a chelate.

### FIGURES

**Figure 1** illustrates the SPECT/CT imaging of [¹¹¹In]In-CB[7]-DOTA at 1 h, 4 h and 24 h p.i. in two mice (top), and ex vivo biodistribution of [¹¹¹In]In-CB[7]-DOTA 24 h p.i. (bottom), according to **example 6.**
**Figure 2** shows the SPECT/CT imaging of [¹¹¹In]In-CB[7]-DOTA⊂trastuzumabadamantanamine 72 h p.i. (left), and ex vivo biodistribution of [¹¹¹In]In-CB[7]-DOTA⊂trastuzumab-adamantanamine (right), according to **example 6.**

### EXAMPLES

### Example 1: Synthesis of compounds of the invention

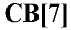

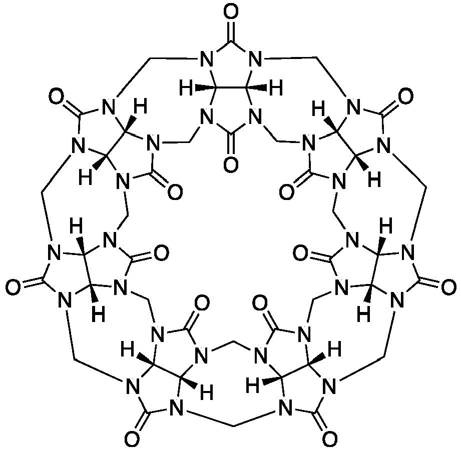

Glycoluril (11.5 g, 80.7 mmol) was dissolved in a mixture of sulfuric acid 9 M (60 mL) and formaldehyde (20 mL) previously cooled to 0 °C. The reaction was stirred 3 h at 95 °C. The mixture was poured into ultrapure H₂O/acetone (1:4) and a white precipitate was formed. The precipitate was filtered and washed twice with 250 mL of H₂O/acetone (1:4). The product was dried under vacuum overnight. CB[7] was obtained as a white powder (1.8 g, 7 %).
¹H NMR (500 MHz, D₂O, 298 K): δ (ppm) = 5.79 (d, 14H), 5.57 (s, 14H), 4.27 (d, 14H). MS: m/z calculated for C₄₆H₅₆N₃₀O₁₄S₂ [M + cystamine + 2H]²⁺/2: 658.6 found 658.9.

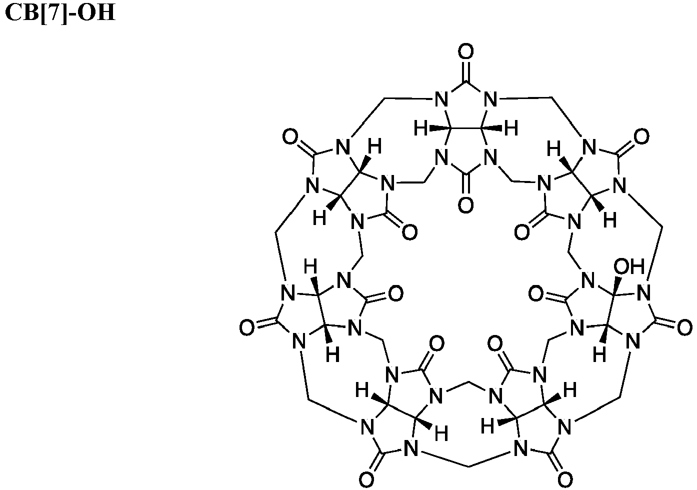

CB[7] (500 mg, 430 mmol) was dissolved in ultrapure water (50 mL). K₂S₂O₈ (116 mg, 430 mmol) and K₂SO₄ (450 mg, 2,58 mol) were added. The mixture was stirred overnight under argon at 85 °C. Purification by flash chromatography on a PS-DVB column (eluent: ultrapure H₂O) afforded CB[7]-OH as a white powder (67 mg, 13 %).
¹HNMR (500 MHz, D₂O, 298 K): δ (ppm) = 5.86 (d, 2H), 5.80 (d, 10H), 5.60 (s, 2H), 5.51 (s, 10H), 5.46 (d, 2H), 5.29 (s, 1H), 4.52 (d, 2H), 4.26 (d, 2H), 4.21 (d, 10 H).
MS: m/z calculated for C₄₆H₅₆N₃₀O₁₅S₂ [M + cystamine + 2H]²⁺/2: 666.6, found 667.0.

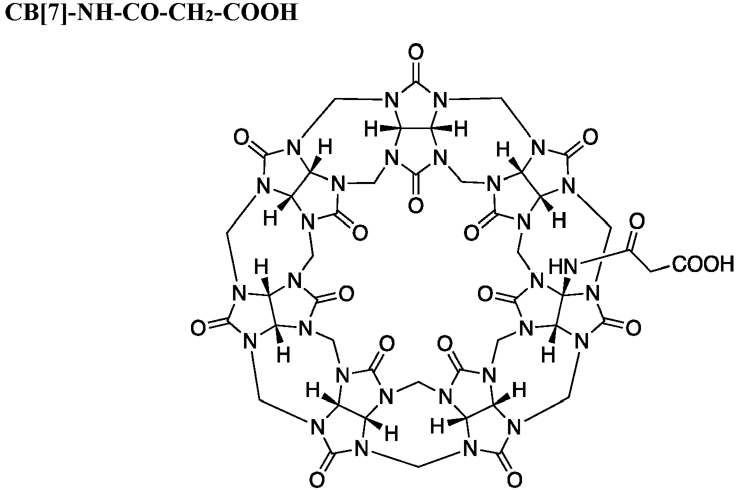

CB[7]-OH (10 mg, 8.5 µmol) and dry 3-cyanopropanoic acid (216 mg, 2.55 mmol) were dissolved in methane sulfonic acid (344 µL). The mixture was sonicated 15 min. Trifluoromethanesulfonic acid (120 µL) was then added dropwise. The reaction was incubated 4.5 hours at 50°C. After cooling to room temperature, a 9:1 acetone/water mixture (12 mL) was added to the reaction and the resulting suspension was centrifugated (4000 rpm, 15 min, 4°C). This procedure was repeated three times. The precipitate was put under vacuum overnight, solubilized in water (1 mL) and freeze-dried to afford the product as a yellow solid (11.5 mg).
MS: m/z calculated for C₄₉H₅₉N₃₁O₁₇S₂ [M + cystamine + 2H]²⁺/2: 708.7 found 709.3.

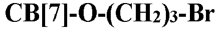

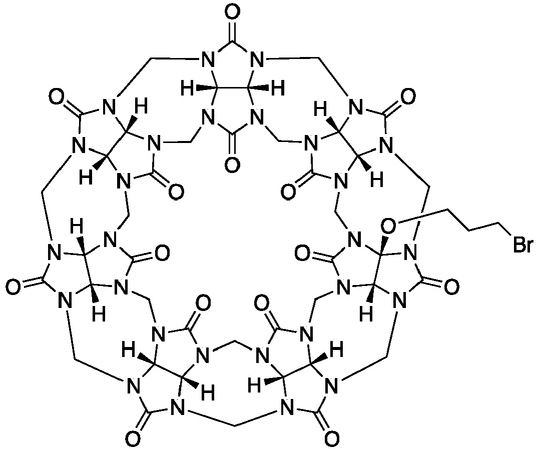

CB[7]-OH (10 mg, 8.5 µmol) and 3-bromopropanol (354 mg, 2.54 mmol) were dissolved in methane sulfonic acid (480 µL). The mixture was sonicated 15 min. Trifluoromethanesulfonic acid (120 µL) was then added dropwise. The reaction was incubated 5 hours at 50°C. After cooling to room temperature, a 8.3:1 acetone/water mixture (9.3 mL) was added to the reaction and the resulting suspension was centrifugated (4000 rpm, 15 min, 4°C). This procedure was repeated once using acetone 100% (8 mL). The precipitate was put under vacuum overnight, solubilized in water (1 mL) and freeze-dried to afford the product as yellow solid (9.0 mg).
MS: m/z calculated for C₄₉H₆₁BrN₃₀O₁₅S₂ [M + cystamine + 2H]²⁺/2: 726.2 found 726.7.

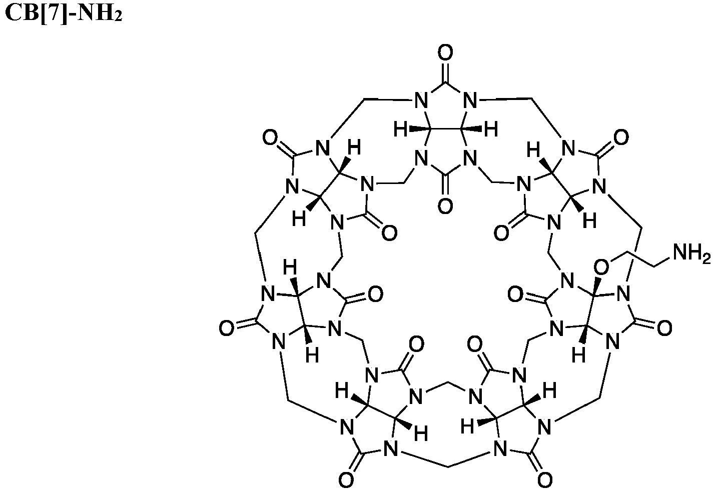

CB[7]-OH (30 mg, 25 µmol) and ethanolamine hydrochloride (745 mg, 7.6 mmol) were dissolved in methane sulfonic acid (1 mL). The mixture was sonicated 15 min. Trifluoromethanesulfonic acid (350 µL) was then added dropwise. The reaction was incubated 6 hours at 37 °C in a thermomixer (600 rpm). After cooling to room temperature, acetone (30 mL) was added to the reaction and the mixture was centrifuged (4000 rpm, 15 min), this procedure was repeated three times. Precipitate was put under vacuum overnight. Purification by semi-preparative HPLC on a Hypercarb column (eluents: H₂O + 0.1 % TFA, MeCN) afforded CB[7]-NH₂ as a white powder (21 mg, 68 %).
MS: m/z calculated for C₄₈H₆₁N₃₁O₁₅S₂ [M + cystamine + 2H]²⁺/2: 688.2 found 688.5.

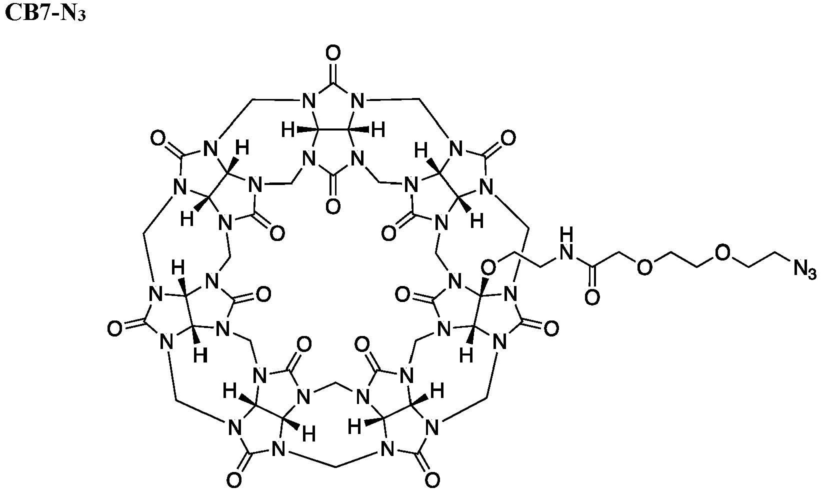

N₃-PEG₂-COOH (13 mg, 7 µmol) was dissolved in dry DMF (200 µL). DIPEA (24 µL, 14 µmol) and TSTU (23 mg, 7.6 µmol) were added. The mixture was stirred 30 min at room temperature in order to form the NHS-ester. CB[7]-NH2 (24 mg, 20 µmol) was dissolved in dry DMF (200 µL) with DIPEA (7 µL, 40 µmol) and directly added to the reaction mixture containing the activated ester. The mixture was stirred 10 min at room temperature. Purification by semi-preparative HPLC on a Hypercarb column (eluents: H₂O + 0.1 % TFA, MeCN) afforded CB[7]-N₃ as a white powder (4 mg, 15 %).
MS: m/z calculated for C₅₄H₇₀N₃₄O₁₈S₂ [M + cystamine + 2H]²⁺/2: 773.7 found 773.6.

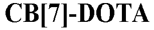

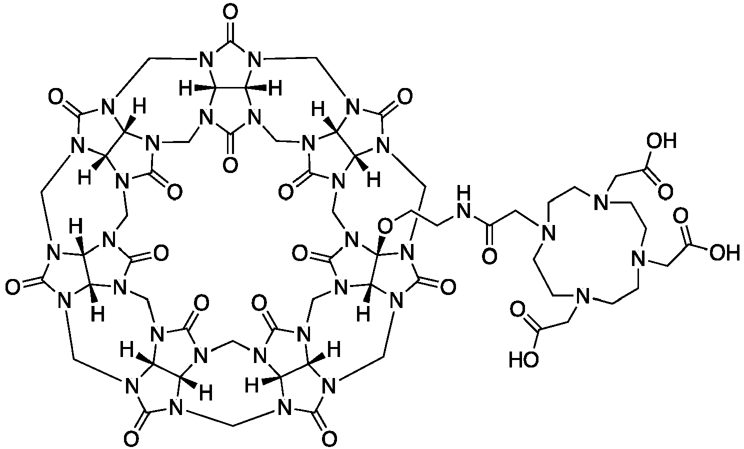

CB[7]-NH₂ (7.9 mg, 6.5 µmol) was dissolved in borate buffer (1 M, pH 8.33). DOTA-NHS (9.7 mg, 19.4 µmol) was then added and the pH was adjusted to pH 9 with NaOH 1 M. Reaction was stirred 15 min at room temperature. Purification by semi-preparative HPLC on a Hypercarb column (eluents: H₂O + 0.1 % TFA, ACN) afforded CB[7]-DOTA as a white powder (4 mg, 39 %).
MS: m/z calculated for C₆₄H₈₇N₃₅O₂₂S₂ [M + cystamine + 2H]²⁺/2: 881.4 found 881.1.

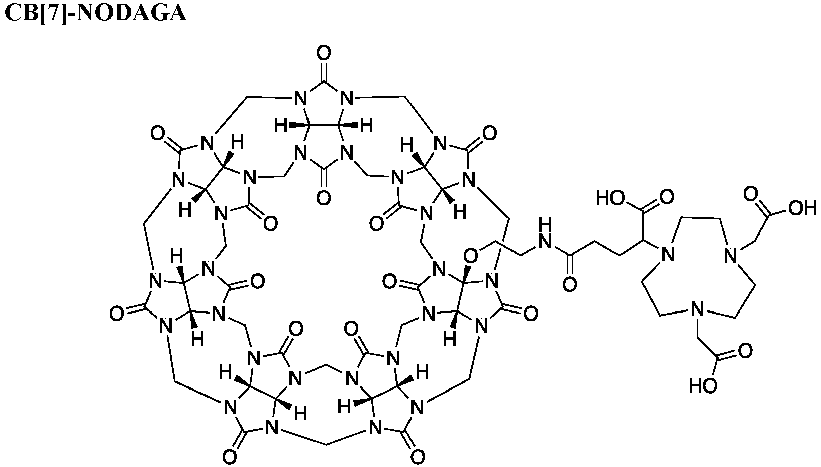

CB[7]-NH₂ (10 mg, 8 µmol) was dissolved in carbonate buffer (0.2 M, pH 9.10). NODAGA-NHS (18 mg, 24 µmol) was added and pH was adjusted to pH 9 with NaOH 1M. The mixture was stirred 15 min at room temperature. Purification was done by semi-preparative HPLC on a Hypercarb column (eluents: H₂O + 0.1 % TFA/ACN). CB[7]-NODAGA was obtained as a white powder (5 mg, 13 %).
MS: m/z calculated for C₆₄H₈₄N₃₄O₂₂S₂ [M + cystamine + 2H]²⁺/2: 866.8 found 867.0.

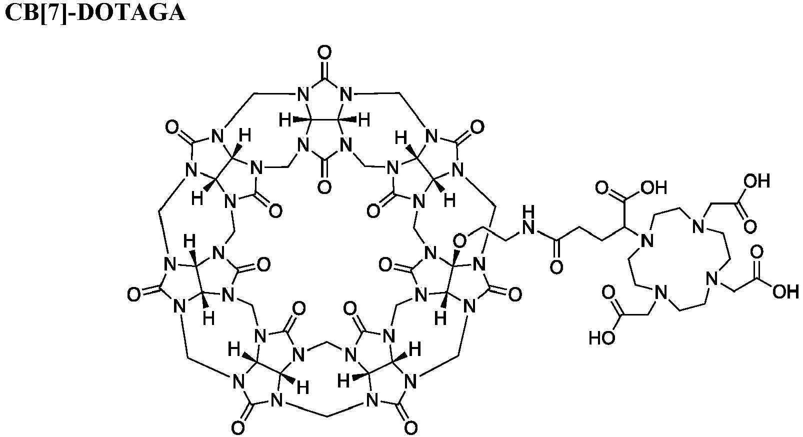

CB[7]-NH₂ (5 mg, 4 µmol) was dissolved in borate buffer (0.1 M, pH 8.9). DOTAGA anhydride (5.5 mg, 12 µmol) was added and pH was adjusted to pH 9 with NaOH 1M. The mixture was stirred 6 hours at room temperature. Purification was done by semi-preparative HPLC on a Hypercarb column (eluents: H₂O + 0.1 % TFA/ACN). CB[7]-DOTAGA was obtained as a white powder (+ TFA salts, 11 mg).
MS: m/z calculated for C₆₃H₇₉N₃₃O₂₄ [M + 2H]²⁺/2: 840.8 found 840.8.

### Example 2: Preparation of supramolecular conjugates of the invention based on trastuzumab, as targeting agent, notably for use in the treatment of breast cancer

Trastuzumab, a monoclonal antibody used to treat breast cancer and stomach cancer, specifically used for cancer that is HER2 receptor positive, was deglycosylated using PNGase F. 5 µL (50 U) of PNGase F was added to 1 mg of trastuzumab (in solution at 11.5 g/L in PBS 0.01 M pH 7.4). The mixture was incubated overnight at 37°C in a thermomixer (1000 rpm). PNGase F was removed from the mixture by ultrafiltration on an Amicon Ultracel-50 kDa (Merck-Millipore) and the purified solution was centrifuged (4000 rpm, 15 min, 4 °C) to concentrate the product at 9.3 g/L.

Adamantanamine-PEG₅-NH₂ (60 equivalents, 6.4 µL of a 60 mM stock solution in PBS 0.01 M pH 7.4) was added to 930 µg of deglycosylated trastuzumab followed by addition of 6.6 µL of mTGase (1.86 U, 2U/mg of protein). Mixture was incubated overnight at 37 °C on a Thermomixer (1000 rpm) and analysed by mass spectrometry. Trastuzumab-adamantanamine was purified by FLPC on an Äkta Pure 25 M chromatography system (GE Healthcare Life Sciences) equipped with a HiTrap protein A column, using AcOH buffer (25 mM, pH 3.12) as eluant. Buffer exchange was then performed with PBS 0.01 M pH 7.4 and the product was obtained at a concentration of 8.6 g/L.

CB[7]-DOTA (according to **example 1**, 2 equivalents with regard to the antibody, 1 mM in H₂O ultrapure) was added to Trastuzumab-adamantanamine (8.6 g/L in PBS 0.01 M pH 7.4). The mixture was incubated 15 min at 37°C on a thermomixer (1000 rpm). After 15 min, analyses were conducted by high-resolution ESI-mass spectrometry. High-resolution mass spectrometry analyses were recorded on a Orbitrap Exploris 240 mass spectrometer (Thermo Scientific) equipped with an electrospray ionization source (H-ESI II). Spectra were deconvoluted on BioPharma Finder software (Thermo Scientific). The conjugate trastuzumab-adamantanamine⊂CB[7]-DOTA was observed upon deconvolution after 15 minutes of incubation. The main product presents a degree of labelling (DOL) of 2 (two CB[7]-DOTA molecules are bound to one molecule of trastuzumab-adamantamine) showing that the supramolecular assembly takes place rapidly, under biologically relevant conditions, with near quantitative efficiency.

### Example 3: Effect of the conjugation according to the invention on trastuzumab affinity for its antigen HER2

The affinity of trastuzumab derivatives for hHER2 was studied by biolayer interferometry (BLI) on a Sartorius Octet^{®} R2 system. All experiments were performed at 30 °C with 1000 rpm shaking. High Precision Streptavidin 2.0 (Sartorius) biosensors were employed. hHER2 and trastuzumab derivatives were used as the ligand and analytes respectively. PBS was used as control. hHER2 was diluted in PBS to obtain a concentration of 20 nM. Biosensors were first dipped into wells containing PBS during 10 min. The loading time of hHER2 on the biosensors was 300 s. Wash of the biosensors with PBS during 120 s was performed before association step. Association time of trastuzumab to hHER2 was 90 s, dissociation time was 300 s in PBS.

Raw sensorgrams were fitted using a 1:2 bivalent analyte model in Octet^{™} data analysis HT software v 12.2. Data were corrected with an alignment of the Y axis to the average of baseline step, an alignment of the inter-step to the association step and a Savitzky-Golay fittering.

The effect of the conjugation on trastuzumab's ability to bind to HER2 was assessed through biolayer interferometry experiments (BLI) on recombinant biotinylated human hHER2 immobilized on a biosensor. Constants of dissociation of 0.85 ± 0.03, 0.29 ± 0.01, 0.36 ± 0.01 and 0.11 ± 0.02 nM were obtained for native trastuzumab, deglycosylated trastuzumab, trastuzumab-adamantanamine, and trastuzumab-Adamantanamine⊂DOTA-CB[7] (**example 2**), respectively. For reference, trastuzumab site-specifically, covalently labeled with H₂N-PEG₄-DOTA (trastuzumab-DOTA) using microbial transglutaminase, showed a K_{d} of 0.52 ± 0.02 nM in the same assay. Thus, the supramolecular conjugation of DOTA-CB[7] to trastuzumab has a negligeable effect of trastuzumab ability to bind recombinant hHER2.

### Example 4: Radiolabeling of trastuzumab-adamantanamine with CB[7]-DOTA and indium-111

45.45 µL of a ¹¹¹InCl₃ solution at 660.07 MBq/mL in HCl was added to 0.81 µg of CB[7]-DOTA (in solution at 0.1 mM in H₂O milliQ). 4.55 µL (0.1xV¹¹¹InCl₃) of AcONH₄ 1 M buffer was added to compensate the acidity of the ¹¹¹InCl₃ solution. The mixture was incubated 30 min at 90 °C. Radiochemical purity of the radioconjugate was measured by radioTLC. Radiochemical yield was >99 %. It is worth noting that the complexation was performed at 90°C, demonstrating the excellent chemical stability of CB[7]-DOTA.

After 30 min, the mixture was cooled to room temperature and 0.5 equivalents of trastuzumab-adamantanamine (8.6 g/L in PBS 0.01 M pH 7.4) was added. The mixture was incubated 5 minutes at room temperature, and near quantitative supramolecular association was observed by radio-SEC-HPLC. The complex obtained showed good stability in human plasma, with 78% integrity of the radioimmunoconjugate after 7 days of incubation at 37°C.

### Example 5: Radiolabeling of trastuzumab-adamantanamine with CB[7]-DOTA and lutetium-177

CB[7]-DOTA (according to **example 1**) was similarly labelled with lutetium-177.

3.62 µL of a ¹⁷⁷LuCl₃ solution at 8287.29 MBq/mL in 0.05 N HCl was added to 0.40 µg of CB[7]-DOTA (in solution at 0.1 mM in H₂O milliQ). 10.14 µL (2.8xV¹¹¹LuCl₃) of AcONH₄ 0.4 M buffer was added to compensate the acidity of the ¹⁷⁷LuCl₃ solution. The mixture was incubated 30 min at 90 °C. Radiochemical purity of the radioconjugate was measured by radioTLC. Radiochemical yield was >99 %.

After 30 min, the mixture was cooled to room temperature and 0.5 equivalents of trastuzumab-adamantanamine (8.6 g/L in PBS 0.01 M pH 7.4) was added. The mixture was incubated 5 minutes at room temperature, and near quantitative coupling was observed by radio-SEC-HPLC. The complex obtained showed good stability in human plasma with 80% integrity of the radioimmunoconjugate after 7 days.

### Example 6: In vivo experiments (SPECT imaging with indium-111)

In order to induce tumor growth, 5 million of SKOV3 cells 2:1 in Matrigel were injected subcutaneously to Balb/c nude mice. Tumor sizes after 1,5 weeks were between 85 mm³ - 850 mm³ (most between 100-300 mm³).

After 15 days, 15 MBq of [¹¹¹In]In-CB[7]-DOTA was administered (intraveneous injection in the tail vein). SPECT/CT imaging was performed 1-, 4- and 24-hours post-injection. After 24 hours, mice were sacrificed and a biodistribution study (n=4) was performed.

In parallel, 14 MBq of [¹¹¹In]-CB[7]-DOTA⊂trastuzumab-adamantanamine (**example 4**) was injected to mice. SPECT/CT imaging was performed 72-hours post-injection. After 72 hours, mice were sacrificed and biodistribution was performed (n=4).

In a control group ("blocking"), 14 MBq of [¹¹¹In]-CB[7]-DOTActrastuzumab-adamantanamine (**example 4**) was injected to mice, along with an excess of unmodified trastuzumab.

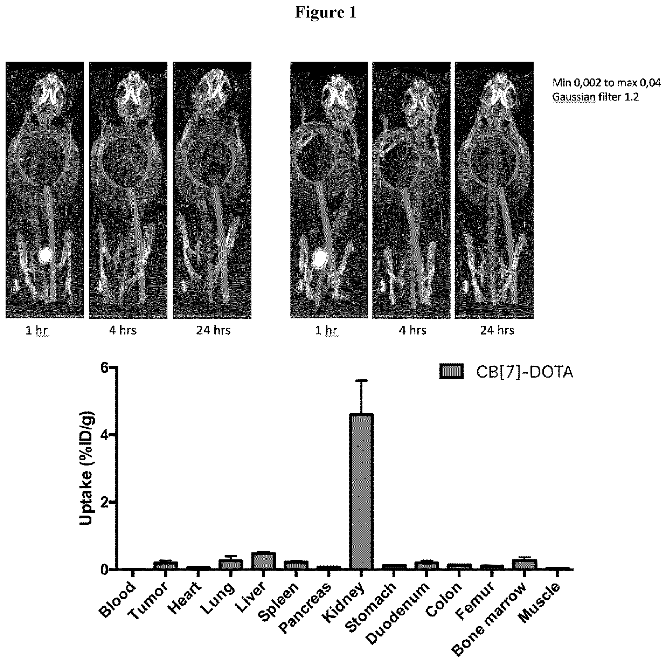

An accumulation of radioactive signal in kidneys and the bladder (urine) was observed after 1h p.i. by SPECT-CT showing that [¹¹¹In]In-CB[7]-DOTA displays a rapid renal elimination (**figure 1**). Most of the radioactive signal was concentrated in the bladder at 1h p.i. Residual activity in the kidneys decreased over 24 h. No significant accumulation of radioactivity was observed in the other organs in the SPECT-CT images.

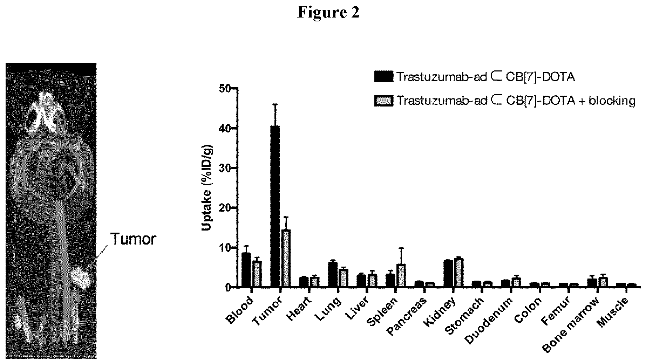

A strong accumulation of the radioactivity in the tumor was observed by SPECT-CT at 72h p.i. upon administration of [¹¹¹In]-CB[7]-DOTA⊂trastuzumab-adamantanamine. These results were confirmed by the biodistribution study (with 40 %ID/g in the tumor). This biodistribution profile is typical the one obtained with ¹¹¹In-radiolabelled trastuzumab derivatives. The uptake of the signal in the tumor was significantly decreased in mice cotreated with an excess of unlabelled trastuzumab (blocking group) demonstrating that the tumor uptake is specific and mediated by trastuzumab binding to its target antigen HER2 (**figure 2**). Thus, the conjugation of CB[7]-DOTA-¹¹¹In to trastuzumab by supramolecular chemistry does not alter its targeting and biodistribution properties. The supramolecular interaction between CB[7]-DOTA-¹¹¹In and trastuzumab-adamantanamine appears stable in vivo, at least up to 72h.

## Claims

1. A compound of following formula (I): 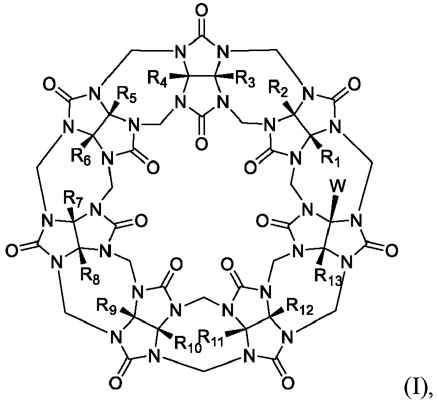 wherein:
W is of following formula (A):
--X-L-(Y)ᵢ-Z (A),
R₁, R₂, R₃, R₄, R₅, R₆, R₇, R₈, R₉, R₁₀, R₁₁ and R₁₂ are identical and are:
- H;
- OH; or
- a methyl group;
R₁₃ is:
- H;
- OH;
- a (C₁-C₆) alkyl group
- a aryl group, in particular a phenyl;
- a O-(C₁-C₁₂)-alkyl, O-(C₂-C₁₂)-alkenyl, or O-(C₂-C₁₂)-alkynyl group, said group being optionally interrupted by at least one atom or group chosen from -O-, -NR-, -S-, and/or substituted, notably terminally substituted, by a T group chosen from halogens, in particular -Cl; ORₐ; and NRₐR_{b}; Rₐ and R_{b} being independently chosen from Hand (C₁-C₁₂)-alkyl groups;
i is at each occurrence independently chosen from 0 and 1;
X is chosen from O, CH₂, NHC(=O), and NH;
L is a linear or branched (C₁-C₂₄)-alcane diyl group, optionally interrupted by at least one atom or group chosen from -O-, -NR-, -S-, -C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-; -C(R)=C(R')-, -C=C-, and arene diyl, in particular benzene diyl, or by a group of the following formula: 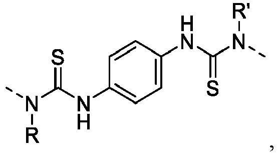
Y is chosen from -O-, -NR-, -S-, -C(=O)-, -O-C(=O)-, -C(=O)-O-, -NR-C(=O)-, - C(=O)-NR-, -NR-C(=O)-NR'-, -NR-C(=O)-O-, -O-C(=O)-NR'-; -C(R)=C(R')-, and -C=C-; -NR-C(=S)-NR'- , and groups of one of the following formulae: 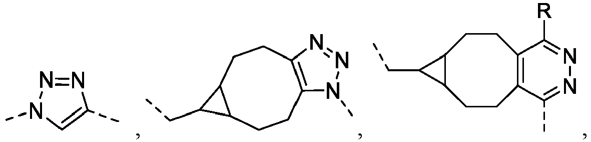 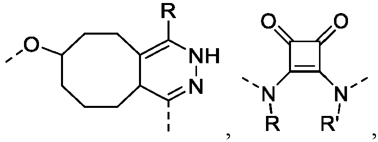 in particular 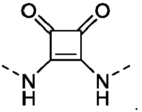
R and R' are at each occurrence independently chosen from H and C₁-C₆-alkyl groups;
Z is a chelator.

2. The compound according to claim 1, wherein:
- all of the R₁-R₁₃ groups are H; or
- R₁₃ group is not H, R₁₃ being in particular OH or a (C₁-C₆) alkyl group, the R₁-R₁₂ groups being H.

3. The compound according to any one of the preceding claims, wherein W is of following formula (A₁): 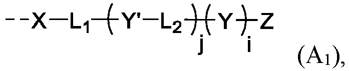 wherein:
X, Y, Z, and i are as defined in claim 1;
j is at each occurrence independently chosen from 0 and 1;
L₁ and L₂ are at each occurrence independently a linear or branched (C₁-C₁₂)-alcane diyl group, optionally interrupted by at least one atom or group chosen from -O-, -NR-, -S-, -C(R)=C(R')-, and -C=C-:
Y' is chosen from -C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-; -C(R)=C(R')-, -C≡C-, and by groups of one of the following formulae: 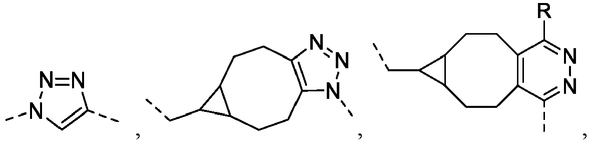 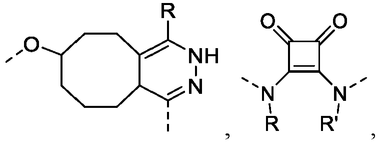 in particular 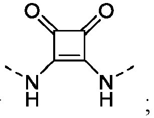
R and R' are at each occurrence independently chosen from H and linear or branched C₁-C₆-alkyl groups.

4. The compound according to any one of the preceding claims, being of the following formula (Ia):

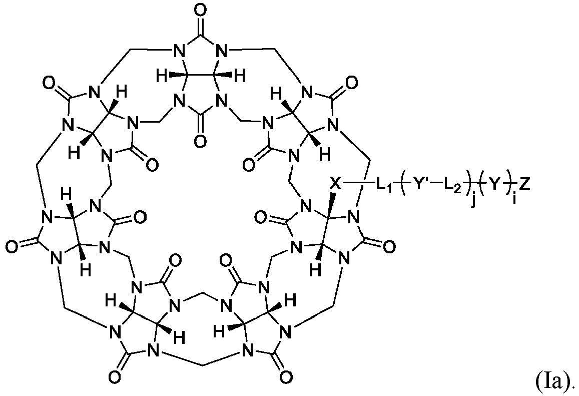

5. The compound according to any one of the preceding claims, wherein W is of one of the following formulae: 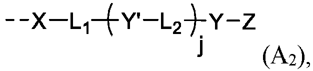 - -X-L₁-Y'-L₂-Z _{(A3)}, --X-L₁-Y'-L₂-Y-Z _{(A4)}, --X-L₁₋Z _{(A5)}, --X-L₁-Y-Z _{(A6)}, wherein X, Y, Y', Z, L₁, L₂ and j are as defined in claim 1 and 3.

6. The compound according to any one of claims 3 to 5, wherein L₁ and/or L₂ are at each occurrence independently:
- a linear (C₁-C₁₂)-alcane diyl group, in particular a linear (C₁-C₆)-alcane diyl group, optionally interrupted by at least one atom -O-;
- a linear (C₁-C₁₂)-alcane diyl group, in particular a linear (C₁-C₆)-alcane diyl group, more particularly a C₂-alcane diyl group;
- -a (CH₂-CH₂-O)ₙ-CH₂-CH₂- group, wherein n ranges from 1 to 7, notably from 1 to 5;
- i is 1 and Y is NH, NHC(=O) or -C(=O)-NH;
- j is 1 and Y' is NHC(=O) or -C(=O)-NH;
- L₁ is a (C₁-C₆)-alcane diyl group, in particular a C₂-alcane diyl group, i is 1, j is 0, and Y is NH or NH-C(=O) or
- L₁ is a (C₁-C₆)-alcane diyl group, in particular a C₂-alcane diyl group, i is 0, j is 1, Y' is NHC(=O) or -C(=O)-NH, and L₂ is a (C₁-C₆)-alcane diyl group, in particular a C₂-alcane diyl group.

7. The compound according to any one of the preceding claims, wherein Z is chosen from DOTA, NODAGA, DOTAGA, DTPA, DFO, NOTA, RESCA, NODA-MPAA, HBED, TETA, CB-TE2A, Macropa, HOPO, TRAP, THP, DATA, NOTP, sarcophagine, FSC, NET A, H4octapa, Pycup, NₓS₄₋ₓ (N4, N2S2, N3S), Hynic, and ^{99m}Tc(CO)₃-chelators.

8. The compound according to any one of the preceding claims, being chosen from the following formulae:

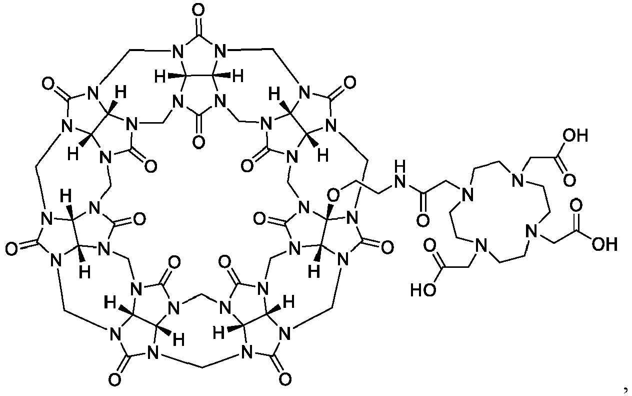

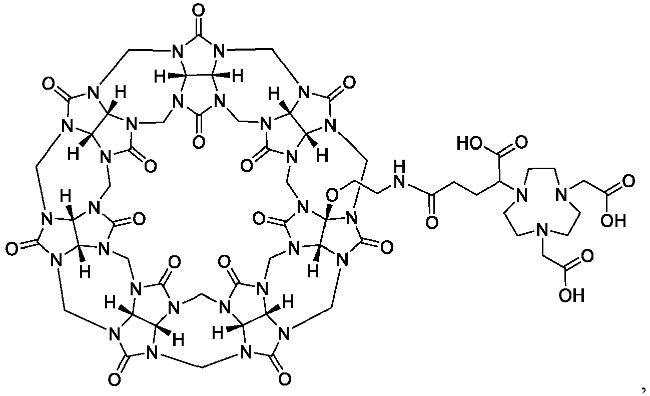

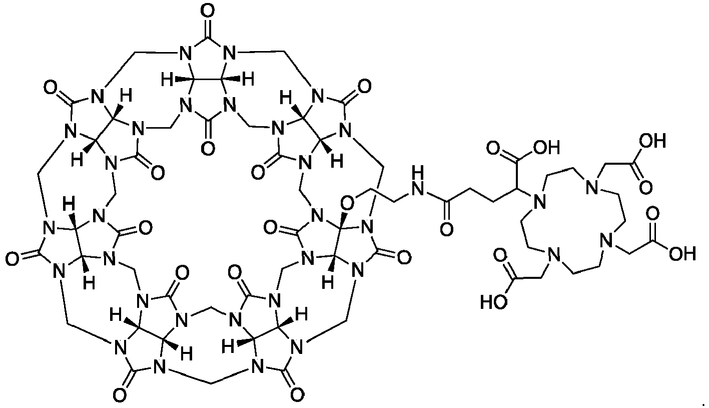

9. A compound of following formula (II): 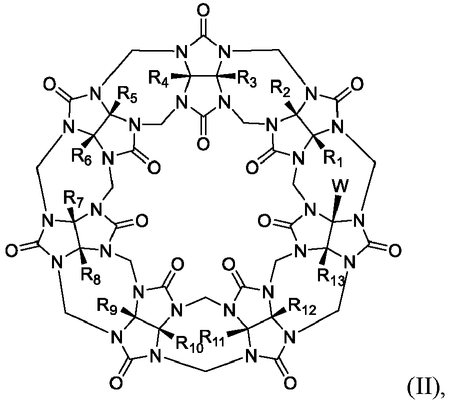 wherein:
W is of following formula (B):
--X-L-(Y)ᵢ-ZM (B),
R₁, R₂, R₃, R₄, R₅, R₆, R₇, R₈, R₉, R₁₀, R₁₁ and R₁₂ are identical and are:
- H;
- OH; or
- a methyl group;
R₁₃ is:
- H;
- OH;
- a (C₁-C₆) alkyl group
- a aryl group, in particular a phenyl;
- a O-(C₁-C₁₂)-alkyl, O-(C₂-C₁₂)-alkenyl, or O-(C₂-C₁₂)-alkynyl group, said group being optionally interrupted by at least one atom or group chosen from -O-, -NR-, -S-, and/or substituted, notably terminally substituted, by a T group chosen from halogens, in particular -Cl; ORₐ; and NRₐR_{b}; Rₐ and R_{b} being independently chosen from Hand (C₁-C₁₂)-alkyl groups;
i is at each occurrence independently chosen from 0 and 1;
X is chosen from O, CH₂, NHC(=O), and NH;
L is a linear or branched (C₁-C₂₄)-alcane diyl group, optionally interrupted by at least one atom or group chosen from -O-, -NR-, -S-, -C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-; -C(R)=C(R')-, -C=C-, and arene diyl, in particular benzene diyl, or by a group of the following formula: 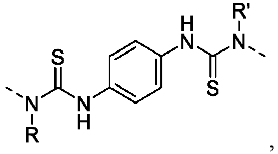
Y is chosen from -O-, -NR-, -S-, -C(=O)-, -O-C(=O)-, -C(=O)-O-, -NR-C(=O)-, - C(=O)-NR-, -NR-C(=O)-NR'-, -NR-C(=O)-O-, -O-C(=O)-NR'-; -C(R)=C(R')-, and -C=C-; -NR-C(=S)-NR'- , and by groups of one of the following formulae: 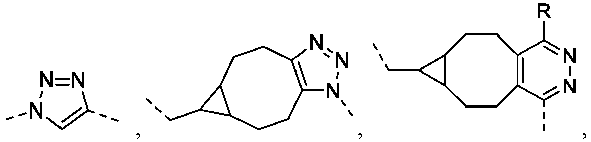 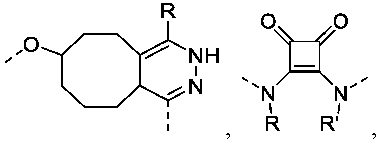 in particular 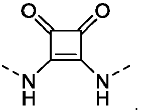
R and R' are at each occurrence independently chosen from H and C₁-C₆-alkyl groups;
Z is a chelator;
M is a radiometal.

10. The compound according to claim 9, wherein M is chosen from⁴³Sc, ⁴⁴Sc, ⁴⁷Sc, ⁵¹Cr, ⁶²Cu, ⁶⁴Cu, ⁶⁷Cu, ⁶⁷Ga, ⁶⁸Ga, ^{81m}Kr, ⁸²Rb, ⁸⁹Zr, ⁹⁰Y, ^{99m}Tc, ¹¹¹In, ^{117m}Sn, ¹⁴⁹Tb, ¹⁵²Tb, ¹⁵³Sm, ¹⁵⁵Tb, ¹⁶¹Tb, ¹⁶⁶Ho, ¹⁷⁷Lu, ¹⁸⁶Re, ¹⁸⁸Re, ²⁰¹Tl, ²⁰³Pb, ²¹²Bi, ²¹²Pb, ²¹³Bi, ²²³Ra, ²²⁵Ac, ²²⁷Th.

11. A conjugate between at least one compound of following formula (II) as defined in claim 9 or 10 and at least one compound (III) comprising a targeting agent, in particular a biomolecule residue, linked, optionally through a linker L', to a binder of cucurbit[7]urils.

12. The conjugate according to claim 11, wherein:
- the targeting agent is a biomolecule, in particular an antibody, more particularly a monoclonal antibody;
- the binder of cucurbit[7]urils is a ferrocene or a polycycloalkane, in particular an adamantane, bicyclo[2.2.2]octane, or a diamantane, said ferrocene or polycycloalkane substituted by at least one amine -NR_{c}R_{d}, ammonium -N⁺R_{c}R_{d}Rₑ, linear or branched (C₁-C₂)-alkyl-NR_{c}R_{d}, or linear or branched (C₁-C₂)-alkyl-N⁺R_{c}R_{d}Rₑ, wherein Rₑ, R_{d} and Rₑ are independently chosen from H and (C₁-C₆)-alkyl groups, and optionally further substituted by at least one hydroxyl, linear or branched (C₁-C₆)-alkyl, linear or branched (C₁-C₆)-alkyl-OH; and/or
- the linker L' is a linear or branched (C₁-C₂₄)-alcane diyl group, optionally interrupted by at least one atom or group chosen from -O-, -NR-, -S-, -C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-; -C(R)=C(R')-, -C=C-, and arene diyl, in particular benzene diyl, or by a group of the following formula: 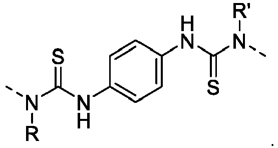
- said linker L' may be linked to said targeting agent by a Y" group chosen from - O-, -NR-, -S-, -C(=O)-, -O-C(=O)-, -C(=O)-O-, -NR-C(=O)-, -C(=O)-NR-, -NR-C(=O)-NR'-, -NR-C(=S)-NR'-, -NR-C(=O)-O-, -O-C(=O)-NR'-; -C(R)=C(R')-, and -C=C-, or by a group of one of the following formulae: 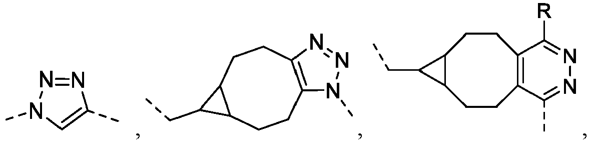 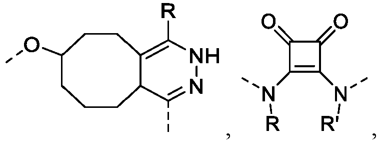 in particular 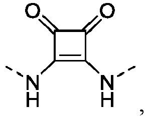
R and R' are at each occurrence independently chosen from H and C₁-C₆-alkyl groups,
L' being for example a PEG.

13. A diagnostic and/or therapeutic composition, comprising a compound of formula (II) as defined in claim 9 or 10, and a pharmaceutically acceptable excipient.

14. The compound of formula (II) as defined in claim 9 or 10 for use as diagnostic or therapeutic agent for detecting, diagnosing or treating cancer, in particular thyroid cancer, neuroendocrine tumour, lung cancer, notably small-cell lung cancer, breast cancer, prostate cancer, notably castration-resistant prostate cancer, bone cancer, in particular bone metastases, non-Hodgkin lymphoma, notably large-cell lymphoma such as low-grade non-Hodgkin lymphoma, transformed low-grade non-Hodgkin lymphoma, diffuse large B-cell non-Hodgkin lymphoma, follicular lymphoma, notably follicular large-cell lymphoma, relapsed non-Hodgkin lymphoma, refractory low-grade non-Hodgkin lymphoma, transformed B-cell non-Hodgkin lymphoma, indolent non-Hodgkin lymphoma, pheochromocytoma, paraganglioma, neuroblastoma, CNS cancer, in particular CNS metastases, small-round-cell tumour, pancreatic cancer, in particular pancreatic ductal adenocarcinoma, colorectal cancer, gastric cancer, head and neck cancer, multiple myeloma, lymphoma, liver cancer, in particular hepatocellular carcinoma, liver metastases, visceral metastases, gastroenteropancreatic neuroendocrine tumours.

15. A combination of a compound of formula (II) as defined in claim 9 or 10, with a compound (III) as defined in claim 11 or 12, for use in the diagnosis or treatment of cancer, in particular thyroid cancer, neuroendocrine tumour, lung cancer, notably small-cell lung cancer, breast cancer, prostate cancer, notably castration-resistant prostate cancer, bone cancer, in particular bone metastases, non-Hodgkin lymphoma, notably large-cell lymphoma such as low-grade non-Hodgkin lymphoma, transformed low-grade non-Hodgkin lymphoma, diffuse large B-cell non-Hodgkin lymphoma, follicular lymphoma, notably follicular large-cell lymphoma, relapsed non-Hodgkin lymphoma, refractory low-grade non-Hodgkin lymphoma, transformed B-cell non-Hodgkin lymphoma, indolent non-Hodgkin lymphoma, pheochromocytoma, paraganglioma, neuroblastoma, CNS cancer, in particular CNS metastases, small-round-cell tumour, pancreatic cancer, in particular pancreatic ductal adenocarcinoma, colorectal cancer, gastric cancer, head and neck cancer, multiple myeloma, lymphoma, liver cancer, in particular hepatocellular carcinoma, liver metastases, visceral metastases, gastroenteropancreatic neuroendocrine tumours, with simultaneous, separate or spread out administration over time

16. A conjugated compound as defined in claim 11 or 12, for use in the diagnosis or treatment of cancer, in particular thyroid cancer, neuroendocrine tumour, lung cancer, notably small-cell lung cancer, breast cancer, prostate cancer, notably castration-resistant prostate cancer, bone cancer, in particular bone metastases, non-Hodgkin lymphoma, notably large-cell lymphoma such as low-grade non-Hodgkin lymphoma, transformed low-grade non-Hodgkin lymphoma, diffuse large B-cell non-Hodgkin lymphoma, follicular lymphoma, notably follicular large-cell lymphoma, relapsed non-Hodgkin lymphoma, refractory low-grade non-Hodgkin lymphoma, transformed B-cell non-Hodgkin lymphoma, indolent non-Hodgkin lymphoma, pheochromocytoma, paraganglioma, neuroblastoma, CNS cancer, in particular CNS metastases, small-round-cell tumour, pancreatic cancer, in particular pancreatic ductal adenocarcinoma, colorectal cancer, gastric cancer, head and neck cancer, multiple myeloma, lymphoma, liver cancer, in particular hepatocellular carcinoma, liver metastases, visceral metastases, gastroenteropancreatic neuroendocrine tumours.
